# FASCICULE DE BREVET EUROPEEN

(11) **EP 0 514 282 B1**
(45) Date de publication et mention de la délivrance du brevet: **10.04.1996**
(21) Numéro de dépôt: 92401344.4
(22) Date de dépôt: 15.05.1992
(51) Int. Cl.: C07C 323/12, C07C 323/41, C07C 327/28, C07C 327/30, A61K 7/06, A61K 7/09

(54) **Nouveaux alkylamino mercaptoalkylamides ou l'un de leurs sels cosmétiquement acceptables, et leur utilisation en tant qu'agents réducteurs, dans un procédé de déformation permanente des cheveux**
Alkylaminomerkaptoalkylamide, für Kosmetika akzeptabele Salze davon, ihre Verwendung als Reduktionsmittel sowie Verfahren zur dauerhaften Verformung von Haaren
Alkylamino mercaptoalkylamides or salts thereof acceptable for cosmetic use, their use as a reducing agent and a process for the permanent deformation of hair

(30) Priorité: 17.05.1991 FR 9106029
(43) Date de publication de la demande: 19.11.1992
(73) Titulaire: L'OREAL, F-75008 Paris (FR)
(72) Inventeur: Junino, Alex, F-93190 - Livry Gargan (FR); Malle, Gérard, F-77580 - Villiers sur morin (FR); Luppi, Bernadette, F-93270 - Sevran (FR)
(74) Mandataire: Stalla-Bourdillon, Bernard

(56) Documents cités:
- EP-A- 0 131 500
- EP-A- 0 432 000
- CHEMICAL ABSTRACTS, vol. 92, 1980, page 566, abrégé no. 163592h, Columbus, Ohio, US; & JP-A-80 000 335 (HAMARI YAKUHIN KOGYO) 05-01-1980
- JOURNAL OF MEDICINAL CHEMISTRY, vol. 29, 1986, pages 2217-2225, Washington, DC, US; J. OIRY et al.: "Synthesis and radioprotective activity of new cysteamine and cystamine derivatives"
- CHEMICAL ABSTRACTS, vol. 66, 1967, page 5382, abrégé no. 55749u, Columbus, Ohio, US; & JP-A-66 019 484 (DAIICHI SEIYAKU)
- EUROPEAN JOURNAL OF MEDICINAL CHEMISTRY, vol. 23, 1988, pages 257-266, Paris, FR; M. FATOME et al.: "Etude de germathiazolidines et de dithioacétals germaniés dérivés de la cystéamine et méthylcystéamine N-substituées: synthèse et activité radioprotectrice"
- CHEMICAL ABSTRACTS, vol. 83, 1975, page 512, abrégé no. 178363x, Columbus, Ohio, US; & JP-A-75 062 932 (DAIICHI SEIYAKU) 29-05-1975
- JOURNAL OF PHARMACEUTICAL SCIENCES, vol. 53, 1964, pages 906-908, Washington, DC, US; W.O. FOYE et al.: "Antiradiation compounds V alpha-amino acid esters of 2-mercaptoethylammine"

## Description

La présente invention a pour objet de nouveaux alkylamino mercaptoalkylamides ou l'un de leurs sels cosmétiquement acceptables et leur utilisation, en tant qu'agents réducteurs, dans un procédé de déformation permanente des cheveux.

La technique pour réaliser la déformation permanente des cheveux consiste, dans un premier temps, à réaliser l'ouverture des liaisons disulfures de la kératine (cystine) à l'aide d'une composition contenant un agent réducteur (étape de réduction), puis, après avoir de préférence rincé la chevelure, à reconstituer dans un second temps lesdites liaisons disulfures en appliquant, sur les cheveux sous tension, une composition oxydante, (étape d'oxydation, dite aussi de fixation) de façon à donner aux cheveux la forme recherchée. Cette technique permet indifféremment de réaliser soit l'ondulation des cheveux, soit leur défrisage ou leur décrêpage.

Les compositions pour réaliser le premier temps d'une opération de permanente se présentent généralement sous forme de lotions, de crèmes, de gels ou de poudres à diluer dans un support liquide et contiennent, en tant qu'agent réducteur, de préférence un mercaptan.

Parmi ces derniers, ceux couramment utilisés sont l'acide thioglycolique et l'acide thiolactique ou un mélange de ces acides ainsi que leurs esters par exemple le monothioglycolate de glycérol ou de glycol.

Ces agents réducteurs sont particulièrement efficaces pour réduire les liaisons disulfures de la kératine notamment l'acide thioglycolique qui peut être considéré comme le produit de référence en permanente, et qui conduit à un taux de réduction d'environ 50 %.

Ces agents réducteurs présentent cependant un inconvénient majeur dans la mesure où ils dégagent de mauvaises odeurs.

En vue d'y remédier, il est généralement fait usage d'un parfum permettant de masquer les odeurs.

Après d'importantes recherches, on a maintenant constaté, de façon tout à fait inattendue et surprenante, qu'en utilisant une nouvelle famille d'alkylamino-mercaptoalkylamides ou leurs sels cosmétiquement acceptables, il était possible de remédier aux inconvénients des agents réducteurs de l'état de la technique.

Les agents réducteurs des compositions, selon l'invention, présentent non seulement l'avantage d'être pratiquement dépourvus d'odeur mais permettent également d'obtenir un rendement, une nervosité et une beauté de frisure supérieurs à ceux obtenus selon l'état de la technique à l'aide par exemple de l'acide thioglycolique.

La présente invention a donc pour objet, à titre de produits industriels nouveaux, les alkylamino-mercaptoalkylamides correspondants à la formule générale (I) suivante : 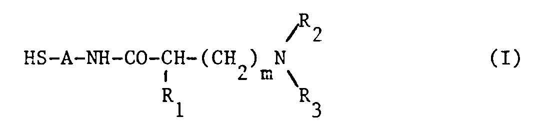
dans laquelle :
A représente le radical divalent -(CH₂)ₙ-,
n étant un nombre entier compris entre 2 et 5, ou le radical divalent -(CH₂)₂-O-(CH₂)₂-
m est égal à 0,1 ou 2
R₁ représente un atome d'hydrogène ou un radical alkyle inférieur, linéaire ou ramifié, ayant de 1 à 5 atomes de carbone,
R₂ et R₃, identiques ou différents, représentent un atome d'hydrogène ou un radical alkyle inférieur, linéaire ou ramifié, ayant de 1 à 4 atomes de carbone, R₂ et R₃ ne pouvant simultanément représenter un atome d'hydrogène,
et les sels desdits composés de formule (I).

Parmi les sels cosmétiquement acceptables des composés de formule (I), ceux particulièrement préférés sont les chlorhydrates, bromhydrates, citrates, oxalates et acétates.

Par radical alkyle inférieur ayant soit de 1 à 4 ou de 1 à 5 atomes de carbone on doit entendre un radical méthyle, éthyle, propyle, isopropyle, butyle, isobutyle, méthyl-2 butyle ou pentyle.

Parmi les composés préférés répondant à la formule générale (I) on peut notamment citer :
- le diméthylamino-2 N-(mercapto-2 éthyl) acétamide,
- le diméthylamino-2 N-(mercapto-3 propyl) acétamide,
- le diméthylamino-2 N-(mercapto-5 pentyl) acétamide,
- le diméthylamino-2 N-[(mercapto-2-éthoxy)-2′éthyl] acétamide,
- le diéthylamino-2 N-(mercapto-2 éthyl) acétamide,
- le diéthylamino-2 N-(mercapto-3 propyl) acétamide,
- le diéthylamino-2 N-(mercapto-5 pentyl) acétamide,
- le diéthylamino-2 N-[(mercapto-2 éthoxy)-2′éthyl] acétamide,
- le méthylamino-2 N-(mercapto-2 éthyl) acétamide,
- le méthylamino-2 N-(mercapto-3 propyl) acétamide,
- le méthylamino-2 N-(mercapto-5 pentyl) acétamide,
- le méthylamino-2 N-[(mercapto-2 éthoxy)-2′éthyl] acétamide,
- l'éthylamino-2 N-(mercapto-2 éthyl) acétamide,
- l'éthylamino-2 N-(mercapto-3 propyl) acétamide,
- l'éthylamino-2 N-(mercapto-5 pentyl) acétamide,
- l'éthylmamino-2 N-[(mercapto-2 éthoxy)-2′éthyl] acétamide,
- l'éthylamino-2 méthylamino-2 N-(mercapto-2 éthyl) acétamide,
- le propylamino-2 N-(mercapto-2 éthyl) acétamide,
- l'isopropylamino-2 N-(mercapto-2 éthyl) acétamide,
- le butylamino-2 N-(mercapto-2 éthyl) acétamide,
- l'éthylméthylamino-2 N-(mercapto-2 éthyl) acétamide,
- le diméthylamino-3 N-(mercapto-2 éthyl) propionamide
- diméthylamino-3 N-(mercapto-3 propyl) propionamide,
- le diméthylamino-3 N-(mercapto-5 pentyl) propionamide,
- le diméthylamino-3 N-[(mercapto-2 éthoxy)-2′éthyl] propionamide,
- le méthylamino-3 N-(mercapto-2 éthyl) propionamide,
- le méthylamino-3 N-(mercapto-3 propyl) propionamide,
- le méthylamino-3 N-(mercapto-5 pentyl) propionamide,
- le méthylamino-3 N-[(mercapto-2 éthoxy)-2′éthyl] propionamide,
- le diméthylamino-2 N-(mercapto-2 éthyl) propionamide,
- le diméthylamino-2 N-(mercapto-3 propyl) propionamide,
- le diméthylamino-2 N-(mercapto-5 pentyl) propionamide,
- le diméthylamino-2 N- [(mercapto-2 éthoxy)-2 éthyl] propionamide,
- le méthylamino-2 N-(mercapto-2 éthyl) propionamide,
- l'éthylamino-2 N-(mercapto-2 éthyl) propionamide,
- l'isopropylamino-2 N-(mercapto-2 éthyl) propionamide,
- le méthylamino-2 N-(mercapto-2 éthyl) butanamide,
- le diméthylamino-4 N-(mercapto-2 éthyl) butanamide,
- le diméthylamino-4 N-(mercapto-3 propyl) butanamide,
- le diméthylamino-4 N-(mercapto-5 pentyl) butanamide,
- le diméthylamino-4 N-[(mercapto-2 éthoxy)-2′éthyl] butanamide,
- le méthylamino-4 N-(mercapto-2 éthyl) butanamide,
- l'éthylamino-4 N-(mercapto-2 éthyl) butanamide,
- le diméthylamino-2 méthyl-3 N-(mercapto-2 éthyl) butanamide,
- le diméthylamino-2 méthyl-4 N-(mercapto-2 éthyl) pentanamide,
- le méthylamino-2 méthyl-4 N-(mercapto-2 éthyl) pentanamide,
- le diméthylamino-2 méthyl-3 N-(mercapto-2 éthyl) pentanamide et
- le méthylamino-2 méthyl-3 N-(mercapto-2 éthyl) pentanamide.

La présente invention a également pour objet le procédé de préparation des alkylamino-mercaptoalkylamides selon l'invention. Ce procédé consiste à faire réagir un aminothiol (1) sur un halogénure d'acide d'un alkylaminoacide (2) de façon à obtenir un thioester de formule générale (II) qui est ensuite réarrangé en alkylamino-mercaptoalkylamide de formule générale (I) par traitement avec une base, selon le schéma réactionnel suivant : 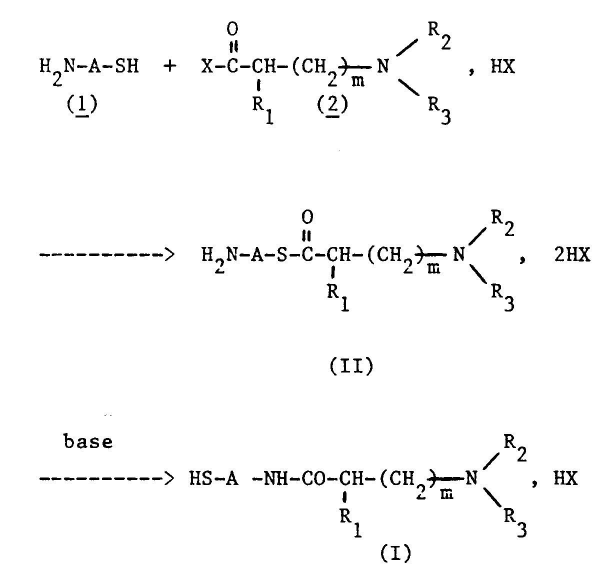
A, R₁, R₂, R₃ et m ont les mêmes significations que ci-dessus pour la formule générale (I), et
X représentant Cl ou Br
Les aminothiols (1) et les halogénures d'acides des alkylaminoacides (2) sont préparés suivant des méthodes connues en soi, les halogénures d'acides étant obtenus par analogie avec la méthode décrite par exemple dans E.Ficher, Chem. Ber.38, 2914-2934, (1905). La réaction entre (1) et (2) est conduite sous atmosphère inerte dans un solvant inerte tel que par exemple le dichlorométhane, le dichloro-1,2 éthane, le trichloro-1,1,1 éthane, le chloroforme, l'acétonitrile, le tétrachlorure de carbone, le toluène, le dioxanne, le tétrahydrofuranne, le diméthoxy-1,2 éthane, la N-méthylpryrrolidone, le diméthylformamide, le diméthylacétamide au un mélange de ces solvants, et suivant le point d'ébullition du solvant à une température comprise entre 40 et 110°C. Le temps réactionnel est généralement inférieur à 5 heures.

Le réarrangement des thioesters de formule générale (II) en alkylamino-mercaptoalkylamides de formule générale (I) est réalisé en milieu aqueux ou hydroalcoolique à température ambiante et sous atmosphère inerte en présence d'au moins un équivalent d'une base telle que l'ammoniaque, les hydroxydes de métaux alcalins ou encore. une amine tertiaire telle que la triéthylamine ou la triéthanolamine.

La présente invention a également pour objet une composition réductrice pour le premier temps d'une opération de déformation permanente des cheveux contenant, dans un véhicule cosmétique approprié, au moins un alkylamino mercaptoalkylamide de formule générale (I) en tant qu'agent réducteur.

Dans les compositions selon l'invention l'agent réducteur de formule générale (I) est généralement présent à une concentration comprise entre 2 et 30% et de préférence entre 5 et 25% en poids par rapport au poids total de la composition réductrice.

Le pH de la composition est de préférence compris entre 4,5 et 11 et plus particulièrement entre 6 et 10 et est obtenu à l'aide d'un agent alcalin tel que par exemple l'ammoniaque, la monoéthanolamine, la diéthanolamine, la triéthanolamine, un carbonate ou bicarbonate alcalin ou d'ammonium, un hydroxyde alcalin ou à l'aide d'un agent acidifiant tel que par exemple l'acide chlorhydrique, l'acide acétique, l'acide lactique, l'acide oxalique ou l'acide borique.

La composition réductrice peut également contenir d'autres agents réducteurs connus tels que par exemple l'acide thioglycolique, le monothioglycolate de glycérol ou de glycol, la cystéamine et ses dérivés acylés C₁-C₄ tels que la N-acétyl cystéamine ou la N-propionyl cystéamine, la cystéine, la N-acétylcystéine, les N-mercaptoalkylamides de sucres tels que le N-(mercapto-2-éthyl)gluconamide, l'acide β-mercaptopropionique et ses dérivés, l'acide thiolactique, l'acide thiomalique, la pantéthéine, le thioglycérol, les sulfites ou les bisulfites d'un métal alcalin ou alcalino-terreux, les N-(mercaptoalkyl)ω-hydroxyalkylamides décrits dans EP 354 835, les N-mono- ou N,N dialkylmercapto 4-butyramides décrits dans EP 368 763, les aminomercapto alkylamides décrits dans EP 403 267 et les dérivés des acides N-(mercaptoalkyl) succinamiques ou des N-(mercaptoalkyl) succinimides décrits dans EP 465 342.

La composition réductrice peut en outre contenir divers ingrédients tels que par exemple des polymères cationiques tels que ceux utilisés dans les compositions des brevets français n° 79.32078 et 80.26421 ou encore des polymères cationiques de type ionène tels que ceux utilisés dans les compositions du brevet français n° 82.17364, des agents adoucissants et notamment des ammonium quaternaires dérivés de la lanoline, des hydrolysats de protéines, des cires, des agents opacifiants, des parfums, des colorants, des tensio-actifs non ioniques ou cationiques, des alcools tels que l'éthanol, le propanol, l'isopropanol, le propanediol-1,2, le butanediol-1,2 ou le glycérol, des agents traitants ou encore des agents Le pénétration tels que l'urée, la pyrrolidone ou la thiamorpholinone.

La composition réductrice selon l'invention peut être également du type exothermique, c'est-à-dire provoquant un certain échauffement lors de l'application- sur les cheveux, ce qui apporte un agrément à la personne qui subit le premier temps de la permanente ou du défrisage.

Le véhicule des compositions selon l'invention est de préférence de l'eau ou une solution hydroalcoolique d'un alcool inférieur tel que l'éthanol, l'isopropanol ou le butanol.

Lorsque les compositions sont destinées à une opération de défrisage ou de décrêpage des cheveux, la composition réductrice est de préférence sous forme d'une crème de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc... On peut également utiliser des liquides ou des gels contenant des agents épaississants tels que des polymères ou des copolymères carboxyvinyliques qui "collent" les cheveux et les maintiennent dans la position lisse pendant le temps de pose.

Selon une forme de réalisation particulière de l'invention, les composés réducteurs de formule générale (I) peuvent être formés in situ au moment de l'emploi à partir de. leurs précurseurs thioesters répondant à la formule générale (II) suivante : 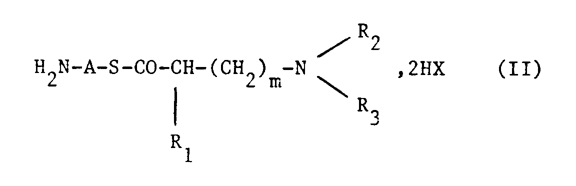
dans laquelle :
A, R₁, R₂, et R₃et m ont les mêmes significations que ci-dessus pour la formule générale (I), et
X est Cl ou Br.

On a en effet constaté qu'en présence d'une base telle que l'ammoniaque, la monoéthanolamine, la diéthanolamine, la triéthanolamine, la soude ou la potasse, ces précurseurs thioesters se transformaient quasi instantanément et quantitativement en thiols correspondants à la formule générale (I).

Selon cette forme de réalisation, le précurseur thioester à l'état solide, de préférence sous forme de poudre, est mélangé au moment de l'emploi à une solution aqueuse basique de pH compris entre 8 et 10, contenant éventuellement divers ingrédients tels que ceux mentionnés ci-dessus.

Le pH de la composition est ensuite ajusté, si nécessaire, à l'aide d'un agent alcalin ou d'un agent acidifiant tels que ceux précédemment mentionnés.

Le présente invention a donc pour objet à titre de produits nouveaux les thioesters répondant à la formule générale (II) ci-dessus en tant que précurseurs des alkylamino mercaptoalkylamides de formule générale (I).

Parmi ces thioesters on peut notamment citer les composés suivants :
- le dichlorhydrate du N,N-diméthyl thioglycinate d'amino-2 éthyle,
- le dichlorhydrate du N,N-diméthyl thioglycinate d'amino-3 propyle,
- le dichlorhydrate du N,N-diméthyl thioglycinate d'amino-5 pentyle,
- le dichlorhydrate du N,N-diméthyl thioglycinate d'(amino-2 éthoxy)-2′éthyle,
- le dichlorhydrate du N,N-diéthyl thioglycinate d'amino-2 éthyle,
- le dichlorhydrate du N,N-diéthyl thioglycinate d'amino-3 propyle,
- le dichlorhydrate du N,N-diéthyl- thioglycinate d'amino-5 pentyle,
- le dichlorhydrate du N,N-diéthyl thioglycinate d'(amino-2 éthoxy)-2′éthyle,
- le dichlorhydrate du N-méthyl thioglycinate d'amino-2 éthyle,
- le dichlorhydrate du N-méthyl thioglycinate d'amino-3 propyle,
- le dichlorhydrate du N-méthyl thioglycinate d'amino-5 pentyle,
- le dichlorhydrate du N-méthyl thioglycinate d'(amino-2 éthoxy)-2′éthyle,
- le dichlorhydrate du N-éthyl thioglycinate d'amino-2 éthyle,
- le dichlorhydrate du N-éthyl thioglycinate d'amino-3 propyle,
- le dichlorhydrate du N-éthyl thioglycinate d'amino-5 pentyle,
- le dichlorhydrate du N-éthyl thioglycinate d'(amino-2 éthoxy)-2′ éthyle,
- le dichlorhydrate du N-éthyl N-méthyl thioglycinate d'amino-2 éthyle,
- le dichlorhydrate du N-propyl thioglycinate d'amino-2 éthyle,
- le dichlorhydrate du N-isopropyl thioglycinate d'amino-2 éthyle,
- le dichlorhydrate du N-butyl thioglycinate d'amino-2 éthyle,
- le dichlorhydrate du diméthylamino-3 thiopropionate d'amino-2 éthyle,
- le dichlorhydrate du diméthylamino-3 thiopropionate d'amino-3 propyle,
- le dichlorhydrate du diméthylamino-3 thiopropionate d'amino-5 pentyle,
- le dichlorhydrate du diméthylamino-3 thiopropionate d'(amino-2 éthoxy)-2′ éthyle,
- le dichlorhydrate du méthylamino-3 thiopropionate d'amino-2 éthyle,
- le dichlorhydrate du méthylamino-3 thiopropionate d'amino-3 propyle,
- le dichlorhydrate du méthylamino-3 thiopropionate d'amino-5 pentyle,
- le dichlorhydrate du méthylamino-3 thiopropionate d'(amino-2 éthoxy)-2′éthyle,
- le dichlorhydrate du N,N-diméthyl thioalaninate d'amino-2 éthyle,
- le dichlorhydrate du N,N-diméthyl thioalaninate d'amino-3 propyle,
- le dichlorhydrate du N,N-diméthyl thioalaninate d'amino-5 pentyle,
- le dichlorhydrate du N,N-diméthyl thioalaninate d'(amino-2 éthoxy)-2′éthyle,
- le dichlorhydrate du N-méthyl thioalaninate d'amino-2 éthyle,
- le dichlorhydrate de N-éthyl thioalaninate d'amino-2 éthyle,
- le dichlorhydrate du N-isopropyl thioalaninate d'amino-2 éthyle,
- le dichlorhydrate du méthylamino-2 thiobutyrate d'amino-2 éthyle,
- le dichlorhydrate du diméthylamino-4 thiobutyrate d'amino-2 éthyle,
- le dichlorhydrate du diméthylamino-4 thiobutyrate d'amino-3 propyle,
- le dichlorhydrate du diméthylamino-4 thiobutyrate d'amino-5 pentyle,
- le dichlorhydrate du diméthylamino-4 thiobutyrate d'(amino-2 éthoxy)-2′éthyle,
- le dichlorhydrate du méthylamino-4 thiobutyrate d'amino-2 éthyle,
- le dichlorhydrate de l'éthylamino-4 thiobutyrate d'amino-2 éthyle,
- le dichlorhydrate du N,N-diméthylamino thiovalinate d'amino-2 éthyle,
- le dichlorhydrate du N,N-diméthylamino thioleucinate d'amino-2 éthyle,
- le dichlorhydrate du N-méthylamino thioleucinate d'amino-2 éthyle,
- le dichlorhydrate du N,N-diméthylamino thioisoleucinate d'amino-2 éthyle,
- le dichlorhydrate du N-méthylamino thioisoleucinate d'amino-2 éthyle,
- le dichlorhydrate du thiopipécolinate d'amino-2 éthyle et
- le dichlorhydrate de l'hexahydroisothionicotinate d'amino-2 éthyle.

Les compositions selon l'invention peuvent être également sous forme dite "auto-neutralisante" ou encore "auto-régulée" et dans ce cas, le. composé de formule générale (I) est associé à au moins un disulfure soit connu pour son utilisation dans une composition réductrice pour permanente auto-neutralisante soit dérivant d'un composé de formule générale. (I) ou de l'un de ses sels et correspondant à la formule générale (III) suivante : 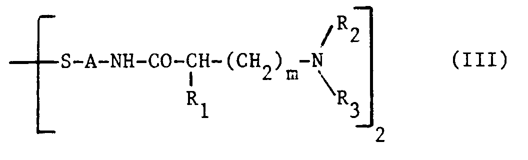
dans laquelle :
A, R₁, R₂ et R₃ et m ont les mêmes significations que ci-dessus pour la formule générale (I) à l'exclusion du composé de formule (III) dans laquelle :
A = -(CH₂)₂-, m = O,
R₁ et R₂ = H et R₃ = -C₂H₅
Le disulfure peut également se présenter sous forme d'un sel cosmétiquement acceptable.

Parmi les disulfures connus, on peut notamment mentionner l'acide dithioglycolique, le dithioglycérol, la cystamine, la N,N′-diacétyl-cystamine, la cystine, la pantéthine, et les disulfures des N-(mercapto-alkyl)ω-hydroxyalkylamides décrits dans EP 354.835 et les disulfures des N-mono ou N,N-dialkylmercapto-4 butyramides décrits dans EP 368.763, les disulfures des aminomercaptoalkylamides décrits dans EP 403 267, et les disulfures des dérivés des acides N-(mercaptoalkyl) succinamiques ou des N-(mercaptoalkyl) succinimides décrits dans EP 465 342.

Parmi les disulfures dérivant d'un composé de formule générale (I) et correspondant à la formule générale (III) on peut notamment citer :
le N,N′-(dithiodiéthanediyl-2,1) bis [diméthylamino-2 acétamide],
le N,N′-(dithiodiéthnediyl-2,1) bis [diméthylamino-2 propionamide]
le N,N′-(dithiodiéthanediyl-2,1) bis [diméthylamino-3 propionamide]
le N,N′-(dithiodiéthanediyl-2,1) bis [diméthylamino-4 butyramide],
le N,N′-(dithiodiéthanediyl-2,1) bis [méthylamino-2 acétamide],
le N,N′-[dithiobis (triméthylène)] bis [diméthylamino-2 acétamide],
le N,N′-[dithiobis (pentaméthylène)] bis [diméthylamino-2 acétamide],
le N,N′-[dithiobis (éthoxy-2 éthyl)] bis [diméthylamine-2 acétamide],
le N,N′-(dithiodiéthanediyl-2,1) bis [méthylamino-3 propionamide],
le N,N′-(dithiodiéthanediyl-2,1) bis [diéthylamino-2 acétamide],
et le N,N′-(dithiodiéthanediyl-2,1) bis [éthylamino-2 acétamide].

Dans les compositions auto-neutralisantes le disulfure est généralement présent en un rapport molaire de 0,5 à 2,5 et de préférence de 1 à 2 par rapport au composé de formule générale (I) ou de ses sels (voir brevet US 3,768,490).

Les disulfures de formule générale (III) sont obtenus par oxydation des composés de formule générale (I) soit à l'air soit en utilisant des oxydants connus comme par exemple l'eau oxygénée en présence éventuellement de sels métalliques tels que par exemple les sels ferreux.

La présente invention a également pour objet un procédé de déformation permanente des cheveux consistant, dans une première étape, à réduire les liaisons disulfures de la kératine par application, pendant environ 5 à 60 min, d'une composition réductrice telle que définie ci-dessus puis dans une seconde étape à reformer lesdites liaisons par application d'une composition oxydante ou éventuellement en laissant agir l'oxygène de l'air.

La présente invention a également pour objet un procédé d'ondulation des cheveux dans lequel on applique une composition réductrice telle que définie ci-dessus sur des cheveux mouillés préalablement enroulés sur des rouleaux ayant de 4 à. 20 mm de diamètre, la composition pouvant éventuellement être appliquée au fur et à mesure de l'enroulage des cheveux ; on laisse ensuite agir la composition réductrice pendant un temps de 5 à 60 minutes, de préférence de 5 à 30 minutes, puis on rince abondamment après quoi on applique, sur les cheveux enroulés, une composition oxydante permettant de reformer les liaisons disulfures de la kératine pendant un temps de pose de 2 à 10 minutes. Après avoir enlevé les rouleaux, on rince abondamment la chevelure.

La composition d'oxydation ou oxydante est du type couramment utilisé et contient comme agent oxydant de l'eau oxygénée, un bromate alcalin, un persel, un polythionate ou un mélange de bromate alcalin et de persel. La concentration en eau oxygénée peut varier de 1 à 20 volumes et de préférence de 1 à 10, la concentration en bromate alcalin de 2 à 12 % et celle en persel de 0,1 à 15 % en poids par rapport au poids total de la composition oxydante. Le pH de la composition oxydante est généralement compris entre 2 et 10. Cette oxydation peut être effectuée immédiatement ou être différée.

La présente invention a également pour objet un procédé de défrisage ou de décrêpage des cheveux dans lequel on applique sur les cheveux une composition réductrice selon l'invention puis l'on soumet les cheveux à une déformation mécanique permettant de les fixer dans leur nouvelle forme, par une opération de lissage des cheveux avec un peigne à larges dents, avec le dos d'un peigne ou à la main. Après un temps de pose de 5 à 60 minutes, en particulier de 5 à 30 minutes, on procède alors à un nouveau lissage puis on rince soigneusement et on applique une composition oxydante ou fixatrice telle que définie ci-dessus, que l'on laisse agir pendant environ 2 à 10 minutes puis on rince abondamment les cheveux.

On va maintenant donner à titre d'illustration et sans aucun caractère limitatif plusieurs exemples de préparation des composés selon l'invention ainsi que des exemples de compositions réductrices selon l'invention et leur utilisation dans un procédé de déformation permanente des cheveux.

### EXEMPLES DE PREPARATION

### EXEMPLE 1 : Préparation du dichlorhydrate du N,N-diméthyl thioglycinate d'amino-2 éthyle

A une suspension de 20g (0,126 mole) de chlorhydrate du chlorure d'acide de la N,N-diméthyl glycine dans 120cm³ d'acétonitrile anhydre, on ajoute, sous atmosphère inerte et sous agitation 14,38g (0,126 mole) de chlorhydrate de cystéamine puis chauffe 3 heures à 75-80°C. Après refroidissement à +5°C, on essore le thioester brut (29,6g) qui est purifié par recristallisation dans le méthanol. On obtient, après séchage sous vide à 40-50°C, 19,5g de dichlorhydrate du N,N-diméthyl thioglycinate d'amino-2 éthyle sous la forme d'un solide blanc de point de fusion 191-3°C.

Le spectre RMN ¹H 250 MHz est conforme à la structure attendue ainsi que le dosage des chlorures (Tr.8,6 méq/g ; Calc.8,5 méq/g).

| Analyse élémentaire : C₆ H₁₄ N₂ O S, 2HCl | | | | | | |
|---|---|---|---|---|---|---|
| | C% | H% | N% | O% | S% | Cl% |
| Calc. | 30,64 | 6,86 | 11,91 | 6,80 | 13,63 | 30,15 |
| Tr. | 30,30 | 6,94 | 10,79 | 6,71 | 13,56 | 30,00 |

### EXEMPLE 2: Preparation du chlorhydrate de diméthylamino-2 N-(mercapto-2 éthyl) acétamide

A une suspension de 11,76g (0,05 mole) de dichlorhydrate du N,N-diméthyl thioglycinate d'amino-2 éthyle, obtenu selon l'exemple 1, dans 100cm³ d'isopropanol, on ajoute goutte à goutte en environ 10 min., à température ambiante sous atmosphère inerte et sous agitation, 41g de solution aqueuse d'ammoniaque à 5%. La solution obtenue est alors évaporée à sec sous pression réduite. On ajoute alors 50cm³ de chloroforme sec et essore sur verre fritté le chlorure d'ammonium. Le filtrat est évaporé à sec sous pression réduite. On obtient un gel qui cristallise lentement à température ambiante. Les cristaux obtenus sont repris par 40cm³ d'acétate d'éthyle anhydre, essorés et séchés sous vide à 50°C.

On obtient ainsi 9,4g de chlorhydrate de diméthylamino-2 N-(mercapto-2 éthyl) acétamide sous la forme d'un solide blanc de point de fusion : 76°C.

Les spectres RMN ¹H 250 MHz et 13C sont conformes à la structure attendue.

| Analyse élémentaire : C₆ H₁₄ N₂ O S, HCl | | | | | | |
|---|---|---|---|---|---|---|
| | C% | H% | N% | O% | S% | Cl% |
| Cal. | 36,27 | 7,61 | 14,10 | 8,05 | 16,14 | 17,84 |
| Tr. | 36,26 | 7,66 | 14,30 | 8,50 | 16,00 | 17,87 |

### EXEMPLE 3 : Préparation du N,N′-(dithiodiéthanediyl-2,1) bis [diméthylamino-2 acétamide]

### a) Chlorhydrate de N,N′-(dithiodiéthanediyl-2,1) bis [diméthylamino-2 acétamide]

A une solution de 2g (0,01 mole) de chlorhydrate de diméthylamino-2 N-(mercapto-2-éthyl) acétamide, obtenu à l'exemple 2, dans 15cm³ d'alcool absolu, on ajoute goutte à goutte 0,55cm³ (5mmoles) d'eau oxygénée à 110 volumes en maintenant la température inférieure à 25°C. On ajoute ensuite une trace de sulfate ferreux et agite 6 heures à température ambiante. Le milieu réactionnel est concentré sous pression réduite. On ajoute 30cm³ de toluène au résidu puis évapore à nouveau à sec.

On obtient, après séchage sous vide à température ambiante, 1,9g de chlorhydrate N,N′-(dithiodiéthanediyl-2,1) bis [diméthylamino-2 acétamide)] sous la forme d'un solide blanc pulvérulent très hygroscopique.

### b) N,N′-(dithiodiéthanediyl-2,1) bis [diméthylamino-2 acétamide]

On dissout 1,58 g (4 mmoles) du composé obtenu en a) ci dessus dans 20 ml d'eau, on ajoute 0,67 g (8 mmoles) de bicarbonate de sodium et on évapore à sec sous pression réduite. Le résidu est repris par 20 ml d'éthanol et le chlorure de sodium est séparé par filtration. Le filtrat est évaporé à sec sous pression réduite. On obtient, après recristallisation dans l'écétone et séchage sous vide à température ambiante 1,13 g de N,N′-(dithiodiéthanediyl-2,1) bis [diméthylamino-2 acétamide] sous la forme d'un solide blanc de point de fusion : 100°C.

Les spectres RMN ¹H 250 MHz est conforme à la structure attendue.

| Analyse élémentaire : | | | | | |
|---|---|---|---|---|---|
| | C% | H% | N% | O% | S% |
| Cal. | 44,69 | 8,13 | 17,37 | 9,92 | 19,89 |
| Tr. | 44,62 | 8,22 | 17,04 | 9,97 | 19,64 |

### EXEMPLE 4 : Préparation du dichlorhydrate du N,N-diéthyl thioglycinate d'amino-2 éthyle

A une suspension de 7g (37 mmoles) de chlorhydrate du chlorure d'acide de la N,N-diéthyl glycine dans 40 cm³ d'acétonitrile anhydre, on ajoute, sous atmosphère inerte et sous agitation, 4,27g (37,5 mmoles) de chlorhydrate de cystéamine puis chauffe 3 H au reflux.

Après refroidissement du milieu réactionnel à +5°C, on essore le thioester brut (9g) qui est purifié dans 20 cm³ d'alcool éthylique à ébullition. On refroidit à + 10°C, essore et obtient après séchage sous vide à 50°C, 6,6g de dichlorhydrate du N,N-diéthyl thioglycinate d'amino-2 éthyle sous la forme d'un solide blanc de point de fusion (décomposition) d'environ 200°C.

Le spectre RMN¹³ C est conforme à la structure attendue ainsi que le dosage des chlorures (tr. 7,64 meq/g ; calc. 7,60 meq/g).

| Analyse élémentaire : C₈H₁₈N₂OS,2HCl | | | | | | |
|---|---|---|---|---|---|---|
| | C% | H% | N% | O% | S% | Cl% |
| Cal. | 36,50 | 7,66 | 10,64 | 6,08 | 12,18 | 26,94 |
| tr. | 36,30 | 7,61 | 10,68 | 6,18 | 12,03 | 27,16 |

### EXEMPLE 5 : Préparation du chlorhydrate de diéthylamino-2 N-(mercapto-2 éthyle) acétamide

A une suspension de 3g (11,4 mmoles) de dichlorhydrate de N,N-diéthyl thioglycinate d'amino-2 éthyle, obtenu à l'exemple 3, dans 20 cm³ d'isopropanol, on ajoute goutte à goutte en environ 20 mn, à température ambiante sous atmosphère d'argon et sous agitation, 8,8 cm³ de solution aqueuse d'ammoniaque à 5%.

La solution obtenue est évaporée à sec sous pression réduite. Le résidu est dispersé dans 40 cm³ d'éthanol, refroidi à 0°C et le chlorure d'ammonium est essoré sur verre fritté. Le filtrat est évaporé à sec sous pression réduite et séché sous vide à 40°C. On obtient 2,4g de chlorhydrate de diéthylamino-2 N-(mercapto-2 éthyl) acétamide sous la forme d'une huile incolore.

Le spectre RMN¹H 250 MHz est conforme à la structure attendue. Le dosage de thiol par iodométrie en milieu acide est également conforme : tr. 4,40 meq/g ; calc. 4,41 meq/g.

### EXEMPLE 6 : Préparation du dichlorhydrate du N-méthyl thioglycinate d'amino 2-éthyle

Un mélange de 63,4g (0,44 mole) de chlorhydrate du chlorure d'acide de la sarcosine et de 50g (0,44 mole) de chlorhydrate de cystéamine dans 200 cm³ d'acétonitrile anhydre est agité 2H au reflux sous atmosphère d'argon.

Après refroidissement à + 5°C, le thioester brut est séparé par décantation du solvant puis purifié par recristallisation dans un mélange éthanol/méthanol. On obtient après séchage sous vide à 40°C, 46g de dichhlorhydrate du N-méthyl thioglycinate d'amino-2- éthyle sous la forme d'un solide blanc de point de fusion (décomposition) d'environ 220°C.

Le spectre RMN ¹³C est conforme à la structure attentue ainsi que le dosage de chlorures (tr. 9,06 meq/g ; calc. 9,04 meq/g)

| Analyse élémentaire : C₅H₁₂N₂OS, 2HCl | | | | | | |
|---|---|---|---|---|---|---|
| | C% | H% | N% | O% | S% | Cl% |
| Cal. | 27,16 | 6,38 | 12,67 | 7,23 | 14,50 | 32,06 |
| tr. | 27,20 | 6,44 | 12,48 | 7,60 | 14,22 | 31,80 |

### EXEMPLE 7 : Préparation du dichlorhydrate du N-éthyl thioglycinate d'aminos-2 éthyle.

Une suspension de 5g (31,6 mmoles) de chlorhydrate du chlorure d'acide de la N-éthyl glycine et de 3,6g (31,7 mmoles) de chlorhydrate de cystéamine dans 50 cm³ d'acétonitrile anhydre est portée au reflux sous agitation et sous atmosptère inerte pendant 2H.

Après refroidissement à +5°C, le solvant est éliminé par décantation et le thioester brut est recristallisé dans un mélange éthanol/méthanol. On obtient après séchage sous vide à 40°C, 3,6g de dichlorhydrate du N-éthyl thioglycinate d'amino-2 éthyle sous la forme d'un solide blanc de point de fusion (décomposition) voisin de 215°C.

Le spectre RMN¹³C est conforme à la structure attendue, ainsi que le dosage des chlorures (tr. 8,6 meq/g ; calc. 8,5 meq/g)

| Analyse élémentaire : C₆H₁₄N₂OS,2HCl | | | | | | |
|---|---|---|---|---|---|---|
| | C% | H% | N% | O% | S% | Cl% |
| Cal. | 30,64 | 6,86 | 11,91 | 6,80 | 13,63 | 30,15 |
| tr. | 30,32 | 6,95 | 11,73 | 6,90 | 13,40 | 29,80 |

### EXEMPLE 8 : Préparation du dichlorhydrate du N-éthyl N-méthyl thioglycinate d'amino-2 éthyle.

Une suspension de 106g (0,616 mole) de chlorhydrate du chlorure d'acide de la N-éthyl N-méthyl glycine et de 70g (0,616 mole) de chlorhydrate de cystéamine dans 400 cm³ d'acétonitrile anhydre est agitée 5H au reflux sous atmosphère inerte.

Après refroidissement à 20°C, le thioester brut est essoré (144,3g) puis purifié par recristallisation dans un mélange éthanol/méthanol 40/60. On obtient, après séchage sous vide à 50°C, 110,5g de dichlorhydrate du N-éthyl N-méthyl thioglycinate d'amino-2 éthyle sous la forme d'un solide blanc de point de fusion (décomposition) d'environ 210°C.

Le spectre RMN ¹³C est conforme à la structure attendue ainsi que le dosage des chlorures (tr. 8,10 meq/g ; calc. 8,02 meq/g).

| Analyse élémentaire : C₇H₁₆N₂OS,2HCl | | | | | | |
|---|---|---|---|---|---|---|
| | C% | H% | N% | O% | S% | Cl% |
| Cal. | 33,74 | 7,28 | 11,24 | 6,42 | 12,87 | 28,45 |
| tr. | 33,74 | 7,30 | 10,78 | 6,72 | 12,36 | 27,90 |

### EXEMPLE 9 : Préparation du chlorhydrate d'éthyméthyl amino-2 N-(mercapto-2 éthyl) acétamide

A une suspension de 30 g (0,12 mole) de dichlorhydrate de N-éthyl N-méthyl thioglycinate d'amino-2 éthyle, obtenu à l'exemple 7, dans 150 cm³ d'isopropanol, agitée sous argon à température ambiante, on ajoute goutte à goutte en environ 30 mn 126,4g de solution aqueuse d'ammoniaque à 5%.

La solution obtenue est évaporée à sec sous pression réduite. On ajoute 100 cm³ d'alcool éthylique et évapore à nouveau à sec sous pression réduite.

Le résidu est repris par 150 cm³ d'éthanol sous agitation, refroidi à 0°C et le chlorure d'ammonium est essoré sur verre fritté. Le filtrat est évaporé à sec et le résidu est repris par 200 cm³ de chloraforme sec. On filtre sur verre fritté pour séparer le chlorure d'ammonium résiduel puis évapore à sec le filtrat et sèche sous vide à 50°C de façon prolongée. On obtient ainsi 24,5g de chlorhydrate d'éthyl méthylamino-2 N-(mercapto-2 éthyl) acétamide sous la forme d'un gel incolore à jaune très pâle.

Les spectres RMN ¹H 400MHz et ¹³C sont conformes à la structure attendue.

Dosage de thiol par iodométrie en milieu acide :
tr. 4,65 meq/g ; calc. 4,70 meq/g.

### EXEMPLE 10 : Préparation du dichlorhydrate du N,N-diméthyl thioglycinate d'amino-3 propyle

Une suspension de 6,5g (41 mmoles) de chlorhydrate du chlorure d'acide de la N,N-diméthyl glycine et de 5,25g (41mmoles) de chlorhydrate de mercapto-3 propylamine dans 40 cm³ d'acétonitrile anhydre est agitée 4H au reflux sous atmosphère d'argon.

Après refroidissement à + 5°C, le thioester brut est essorré puis recristallisé dans un mélange éthanol/méthanol. Après séchage à 40°C, on obtient 5,3g du dichlorhydrate du N,N-diméthyl thioglycinate d'amino-3 propyle sous la forme d'un solide blanc de point de fusion (décomposition) d'environ 215°C.

Le spectre RMN¹³C est conforme à la structure attendue ainsi que le dosage des chlorures (tr. 8,10 meq/g ; calc. 8,02 meq/g).

| Analyse élémentaire : C₇H₁₆N₂OS,2HCl | | | | | | |
|---|---|---|---|---|---|---|
| | C% | H% | N% | O% | S% | Cl% |
| Cal. | 33,74 | 7,28 | 11,24 | 6,42 | 12,87 | 28,45 |
| tr. | 33,68 | 7,18 | 11,17 | 6,19 | 12,92 | 28,25 |

### EXEMPLE 11 : Préparation du dichlorhydrate du N-éthyl thioglycinate d'(amino-2 éthoxy)-2′éthyle

Une suspension de 5g (31,8 mmoles) de chlorhydrate de (mercapto-2 éthoxy)-2′ éthylamine et de 5g (31,6 mmoles) de chlorhydrate du chlorure d'acide de la N-éthyl glycine dans 60 cm³ d'acétonitrile anhydre est portée 3H au reflux sous agitation et sous atmosphère inerte.

Après refroidissement à + 10°C et essorage, le thioester brut est recristallisé dans l'éthanol absolu. On obtient, après séchage sous vide à 50°C, 3,2g de dichlorhydrate de N-éthyl thioglycinate d'(amino-2 éthoxy)-2′ éthyle sous la forme d'un solide blanc de point de fusion (décomposition) d'environ 140°C.

Le spectre RMN¹³ C est conforme à la structure attendue ainsi que le dosage des chlorures (tr. 7,06 meq/g ; calc. 7,03 meq/g).

| Analyse élémentaire : C₈H₁₇N₂O₂S,2HCl | | | | | | |
|---|---|---|---|---|---|---|
| | C% | H% | N% | O% | S% | Cl% |
| Cal. | 34,41 | 7,22 | 10,03 | 11,46 | 11,48 | 25,39 |
| tr. | 34,30 | 7,30 | 10,03 | 11,62 | 11,22 | 25,36 |

### EXEMPLE 12 : Préparation du dichlorhydrate du diméthylamino-4 thiobutyrate d'amino-2 éthyle

Une suspension de 3g (27 mmoles) de chlorhydrate de cystéamine et de 5g (27 mmoles) de chlorhydrate du chlorure d'acide de l'acide diméthylamino-4 butyrique dans 75 ml d'acétonitrile anhydre est portée au reflux pendant 5 H sous agitation et atmosphère inerte.

Après refroidissement à température ambiante et essorage, le thioester brut est recristallisé dans un mélange 8:2 d'éthanol/éther isopropylique. On obtient, après séchage sous vide, 5.87g de dichlorhydrate du diméthylamino-4 thiobutyrate d'amino-2 éthyle sous la forme d'un solide blanc de point de fusion 112°C.

Les spectres RMN¹H et ¹³C sont conformes à la structure attendue ainsi que le dosage des chlorures (tr. 7.72 meq/g ; calc. 7.75 meq/g).

### EXEMPLE 13 : Préparation du dichlorhydrate de l'éthylamino-2 thiobutyrate d'amino-2 éthyle

Une suspension de 3,7g (33 mmoles) de chlorhydrate de cystéamine et de 5 g (33 mmoles) de chlorhydrate du chlorure d'acide de l'acide éthylamino-2 butyrique dans 100 ml d'acétonitrile anhydre est portée au reflux pendant 5 H sous agitation et atmosphère inerte.

Après refroidissement à température ambiante, et essorage, le thioester brut est recristallisé dans un mélange 7:3 d'alcool isopropylique : éther diisopropylique. On obtient après séchage sous vide 5,5 g de dichlorhydrate de l'éthylamino-2 thiobutyrate d'amino-2 éthyle sous la forme d'un solide blanc de point de fusion 121°C.

Les spectres RMN¹H et ¹³C sont conformes à la structure attendue ainsi que le dosage des chlorures (tr. 7.55 meq/g ; calc. 7.60 meq/g).

Les thioesters décrits dans les exemples 1, 4, 6, 7, 8, 10, 11, 12 possèdent une bonne capacité de conservation. Il est donc beaucoup plus commode de les transformer en thiols de façon extemporanée. Ainsi, par addition d'au moins 1 éq. d'agent basique tel que l'ammoniaque, les composés de ces exemples conduisent aux thiols correspondants en solution aqueuse avec un titre supérieur ou égal à 99,8% (Iodométrie en milieu acide).

### EXEMPLES DE COMPOSITIONS

### EXEMPLE A

On prépare selon l'invention une composition réductrice de déformation permanente des cheveux en procédant au mélange des ingrédients suivants :

### A. COMPOSITION REDUCTRICE

| | |
|---|---|
| Chlorhydrate de diméthylamino-2 N-(mercapto-2 éthyl) acétamide | 19 g |
| Monoéthanolamine q.s.p. pH 8,5 Chlorure d'oléocétyl diméthyl hydroxyéthyl ammonium | 0,3 g |
| Conservateur | 0,2 g |
| Parfum | 0,8 g |
| Eau déminéralisée q.s.p. | 100 g |

Cette composition est appliquée sur des cheveux mouillés préalablement enroulés sur des rouleaux. Après avoir laissé agir la composition 15 minutes, on rince abondamment à l'eau puis on applique la composition oxydante suivante :

### B. COMPOSITION OXYDANTE

| | |
|---|---|
| Eau oxygénée | 1,5 g |
| Lauryl éther sulfate de sodium oxyéthyléné avec 2 moles d'oxyde d'éthylène | 3,75 g |
| Acide citrique | 0,5 g |
| Hydrogénophosphate de sodium | 0,5 g |
| Parfum | 0,3 g |
| Eau déminéralisée q.s.p. | 100 g |

On laisse agir la composition oxydante pendant environ 5 minutes, puis on enlève les rouleaux, et rince abondamment la chevelure à l'eau. Après séchage sous casque, les cheveux présentent de belles boucles.

Selon le même mode de réalisation qu'à l'exemple A, on a procédé à une déformation permanente des cheveux à l'aide des compositions réductrices et oxydantes des exemples B à M suivants :

### EXEMPLE B

### A. COMPOSITION REDUCTRICE

| | |
|---|---|
| Chlorhydrate de diméthylamino-2 N-(mercapto-2 éthyl) acétamide | 21 g |
| Acide éthylène diamine tétracétique | 0,2 g |
| Triéthanolamine q.s.p. pH 6,8 Oxyde de Lauramine vendu sous la dénomination de "Aromox DMMCD/W" par la Société AKZO | 2,15 g |
| Parfum | 0,6 g |
| Eau déminéralisée q.s.p. | 100 g |

### B. COMPOSITION OXYDANTE

| | |
|---|---|
| Eau oxygénée | 2,0 g |
| Chlorure d'oléocétyl diméthyl hydroxyéthyl ammonium | 0,3 g |
| Acide phosphorique | 0,5 g |
| p-éthoxyacétanilide (phénacétine) | 0,1 g |
| Hydrolysat de protéines | 0,5 g |
| Parfum | 0,5 g |
| Eau déminéralisée q.s.p. | 100 g |

### EXEMPLE C

### A. COMPOSITION REDUCTRICE

| | |
|---|---|
| Dichlorhydrate du N,N-diméthyl thioglycinate d'amino-2 éthyle | 11 g |
| Solution ammoniacale à 20 % NH₃ | 4,1 g |
| Monoéthanolamine q.s.p. pH 8,0 Hydrogénocarbonate d'ammonium | 2,0 g |
| Cocoamidopropylbétaïne vendu sous la dénomination de "Tegobetaïne HS" par la Société GOLDSCHMIDT | 0,9 g |
| Eau déminéralisée q.s.p. | 100 g |

### B. COMPOSITION OXYDANTE

| | |
|---|---|
| Eau oxygénée | 2,5 g |
| Stannate de sodium | 0,03 g |
| Homopolymère du chlorure de N,N-diméthyl N-2 propényl-2 propéne-1 ammonium (polyquaternium-6) vendu sous la dénomination de "Merquat 100" par la Société MERCK | 1,25 g |
| Acide citrique | 0,6 g |
| Parfum | 0,5 g |
| Eau déminéralisée q.s.p. | 100 g |

### EXEMPLE D

### A. COMPOSITION REDUCTRICE

| | |
|---|---|
| Dichlorhydrate du N,N-diméthyl thioglycinate d'amino-2 éthyle | 17 g |
| N,N'-(dithiodiéthanediyl-2,1) bis [diméthylamino-2 acétamide] | 5,1 g |
| Sel pentasodique de l'acide diéthylène triamine pentacétique | 0,2 g |
| Monoéthanolamine q.s.p. pH 8,9 Huile de castor hydrogénée oxyéthyléné avec 60 moles d'oxyde d'éthylène vendu sous la dénomination de "NIKKOL HCO 60" par la Société NIKKO CHEMICAL | 4 g |
| Homopolymère du chlorure de N,N-diméthyl N-2 propenyl-2 propène-1 ammonium (polyquaternium 7) vendu sous la dénomination de "Merquat 550" par la Société MERCK | 3,8 g |
| Parfum | 0,3 g |
| Eau déminéralisée q.s.p. | 100 g |

### B. COMPOSITION OXYDANTE

| | |
|---|---|
| Eau oxygénée (200 volumes) | 4,8 g |
| Stabilisant | 0,1 g |
| D. Panthenol | 1,0 g |
| α-diméthyl-4'α-(méthyl-4 pentényl-3) cyclohexène-3 méthanol (Bisabolol) vendu sous le nom commercial de "Dragosantol 2/012681" par la Société DRAGOCO | 0,3 g |
| Oxyde de lauryl diméthyl amine | 0,7 g |
| Parfum | 0,4 g |
| Acide lactique q.s.p. pH 3,0 Eau déminéralisée q.s.p. | 100 g |

### EXEMPLE E

### A. COMPOSITION REDUCTRICE

| | |
|---|---|
| Dichlorhydrate du N,N-diméthyl thioglycinate d'amino-2 éthyle | 3,5 g |
| L-cystéine | 5,0 g |
| Monoéthanolamine q.s.p. pH 9,3 Ester stéarique polyéthyléné avec 8 moles d'oxyde d'éthylène vendu sous la dénomination de "Myrj 45" par la Société ICI | 1 g |
| Conservateur | 0,4 g |
| Parfum | 0,3 g |
| Eau déminéralisée q.s.p. | 100 g |

### B. COMPOSITION OXYDANTE

| | |
|---|---|
| Eau oxygénée | 2,5 g |
| Stannate de sodium | 0,02g |
| Lauryl sulfate d'ammonium | 1,5 g |
| Hydrolysat de protéines | 0,6 g |
| Acide citrique | 0,5 g |
| Parfum | 0,4 g |
| Eau déminéralisée q.s.p. | 100 g |

### EXEMPLE F

### A. COMPOSITION REDUCTRICE

| | |
|---|---|
| Dichlorhydrate du N,N-diméthyl thioglycinate d'amino-2 éthyle | 2,5 g |
| N-acétylcystéamine | 6,0 g |
| Solution ammoniacale q.s.p. pH 8,0 Hydrogénocarbonate d'ammonium | 2,0 g |
| Homopolymère du chlorure de N,N-diméthyl N-2 propényl-2 propéne-1 ammonium (polyquaternium-6) vendu sous la dénomination de "Merquat 100" par la Société MERCK | 2,5 g |
| Hydrolysat de collagène | 0,5 g |
| Chlorure d'oléocétyl diméthyl hydroxyéthyl ammonium | 1,0 g |
| Parfum | 0,8 g |
| Eau déminéralisée q.s.p. | 100 g |

### B. COMPOSITION OXYDANTE

| | |
|---|---|
| Eau oxygénée (200 volumes) | 4,8 g |
| Stabilisant | 0,1 g |
| D. Panthenol | 1,0 g |
| α-diméthyl-4'α-(méthyl-4 pentenyl-3) cyclohexène-3 méthanol (Bisabolol) vendu sous la dénomination de "Dragosantol 2/012681" par la Société DRAGOCO | 0,3 g |
| Oxyde de lauryl diméthyl amine | 0,7 g |
| Parfum | 0,4 g |
| Acide lactique q.s.p. pH 3,0 Eau déminéralisée q.s.p. | 100 g |

### EXEMPLE G

### A. COMPOSITION REDUCTRICE

| | |
|---|---|
| Dichlorhydrate du N,N-diméthyl thioglycinate d'amino-2 éthyle | 2,0 g |
| N-propionylcystéamine | 6,5g |
| Monoéthanolamine q.s.p. pH 8,5 Chlorure de cétyl triméthyl ammonium | 1,0 g |
| Parfum | 0,6 g |
| Conservateur | 0,15 g |
| Eau déminéralisée q.s.p. | 100 g |

### B. COMPOSITION OXYDANTE

| | |
|---|---|
| Bromate de sodium | 8 g |
| Triéthanolamine q.s.p. pH 8,0 Phosphate monosodique hydraté (12 H₂O) | 0,3 g |
| Phosphate trisodique hydraté (2 H₂O) | 0,5 g |
| Cocoamidopropylbétaïne vendu sous la dénomination "Tegobétaïne HS" par la Société GOLDSCHMIDT | 1,0 g |
| Parfum | 0,4 g |
| Eau déminéralisée q.s.p. | 100 g |

### EXEMPLE H

### A. COMPOSITION REDUCTRICE

| | |
|---|---|
| Dichlorhydrate du N,N-diméthyl thioglycinate d'amino-2 éthyle | 2,0 g |
| Cystéamine, HCl | 5,2 g |
| Monoéthanolamine q.s.p. pH 9,5 Chlorure de cétyl triméthyl ammonium | 1,0 g |
| Parfum | 0,6 g |
| Conservateur | 0,15 g |
| Eau déminéralisée q.s.p. | 100 g |

### B. COMPOSITION OXYDANTE

| | |
|---|---|
| Bromate de sodium | 8 g |
| Triéthanolamine q.s.p. pH 8,0 Phosphate monosodique hydraté (12 H₂O) | 0,3 g |
| Phosphate trisodique hydraté (2 H₂O) | 0,5 g |
| Cocoamidopropylbétaïne vendu sous la dénomination "Tegobétaïne HS" par la Société GOLDSCHMIDT | 1,0 g |
| Parfum | 0,4 g |
| Eau déminéralisée q.s.p. | 100 g |

### EXEMPLE I:

### A. COMPOSITION REDUCTRICE

| | |
|---|---|
| Dichlorhydrate du N-éthyl N-méthyl thioglycinate d'amino-2 éthyle | 8 g |
| Oxyde de laurylamine (Aromox DMMCD/W vendu par AKZO) | 2 g |
| Acide éthylène diamino tétracétique | 0,15g |
| Parfum Monoéthanolamine qs | pH 8,1 |
| Eau déminéralisée qsp | 100g |

### B. COMPOSITION OXYDANTE

| | |
|---|---|
| Eau oxygénée | 2 g |
| Stannate de sodium | 0,015g |
| Lauryl sulfate d'ammonium | 1,4 g |
| Hydrolysat de protéïnes | 0,6 g |
| Acide citrique | 0,5 g |
| Parfum qs Eau déminéralisée qsp | 100 g |

### EXEMPLE J:

### A. COMPOSITION REDUCTRICE

| | |
|---|---|
| Dichlorhydrate du N-éthyl thioglycinate d'amino 2-éthyle | 19 g |
| Oxyde de laurylamine (Aromox DMMCD/W vendu par AKZO) | 2 g |
| Homopolymère du chlorure de N,N-diméthyl N-2 propényl-2 propène-1 (polyquaternium-6) (MERQUAT 100 vendu par la Société MERCK) | 1,10g |
| Parfum Ammoniaque (20%) qs | pH 8,6 |
| Eau déminéralisée qsp | 100g |

### B.COMPOSITION OXYDANTE

Cette composition est identique à celle de l'exemple I.

### EXEMPLE K :

### A. COMPOSITION REDUCTRICE

| | |
|---|---|
| Dichlorhydrate du N,N diméthyl thioglycinate d'amino-2 éthyle | 12g |
| Ester stéarique polyéthyléné avec 8 moles d'oxyde d'éthylène | 0,85g |
| (MYRJ 45 vendu par la Société ICI) Conservateur | 0,35g |
| Parfum Monoéthanolamine qs | pH 8,8 |
| Eau déminéralisée qsp | 100 g |

### B. COMPOSITION OXYDANTE

Cette composition est identique à celle de l'exemple I.

### EXEMPLE L:

### A. COMPOSITION REDUCTRICE

| | |
|---|---|
| Dichlorhydrate du N-méthyl thioglycinate d'amino-2 éthyle | 20g |
| Oxyde de laurylamine (Aromox DMMCD/W vendu par AKZO) | 2g |
| Conservateur | 0,15 g |
| Parfum Monoéthanolamine qs | pH 7,8 |
| Eau déminéralisée qsp | 100 g |

### B. COMPOSITION OXYDANTE

Cette composition est identique à celle de l'exemple I.

### EXEMPLE M:

### A. COMPOSITION REDUCTRICE

| | |
|---|---|
| Dichlorhydrate du N,N-diéthyl thioglycinate d'amino-2 éthyle | 7,2 g |
| Chlorure d'oléocétyl diméthyl hydroxyéthyl ammonium | 0,3 g |
| Conservateur | 0,15g |
| Parfum Ammoniaque qs | pH 8,0 |
| Eau déminéralisée qsp | 100 g |

### B. COMPOSITION OXYDANTE

Cette composition est identique à celle de l'exemple I.

## Revendications (Revendications pour l'(les) Etat(s) contractant(s) suivant(s): AT, BE, CH, DE, DK, FR, GB, IT, LI, NL, PT, SE)

1. Composés nouveaux, caractérisés par le fait qu'ils répondent à la formule générale (I) suivante : 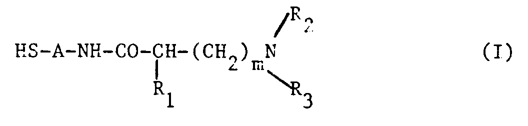 dans laquelle :
A représente le radical divalent -(CH₂)ₙ-,
n étant un nombre entier compris entre 2 et 5, ou le radical divalent -(CH₂)₂-O-(CH₂)₂-
m est égal à 0,1 ou 2
R₁ représente un atome l'hydrogène ou un radical alkyle inférieur, linéaire ou ramifié, ayant de 1 à 5 atomes de carbone,
R₂ et R₃, identiques ou différents, représentent un atome d'hydrogène ou un radical alkyle inférieur, linéaire ou ramifié, ayant de 1 à 4 atomes de carbone, R₂ et R₃ ne pouvant simultanément représenter un atome d'hydrogène, les sels desdits composés de formule (I) ainsi que leurs disulfures correspondants à l'exclusion du disulfure dans lequel :
A = -(CH₂)₂-, m = 0
R₁ et R₂ = H et R₃ = -C₂H₅.

2. Composés selon la revendication 1, caractérisés par le fait qu'ils sont choisis parmi :
- le diméthylamino-2 N-(mercapto-2 éthyl) acétamide,
- le diméthylamino-2 N-(mercapto-3 propyl) acétamide,
- le diméthylamino-2 N-(mercapto-5 pentyl) acétamide,
- le diméthylamino-2 N-[(mercapto-2-éthoxy)-2′éthyl] acétamide,
- le diéthylamino-2 N-(mercapto-2 éthyl) acétamide,
- le diéthylamino-2 N-(mercapto-3 propyl) acétamide,
- le diéthylamino-2 N-(mercapto-5 pentyl) acétamide,
- le diéthylamino-2 N-[(mercapto-2 éthoxy)-2′éthyl] acétamide,
- le méthylamino-2 N-(mercapto-2 éthyl) acétamide,
- le méthylamino-2 N-(mercapto-3 propyl) acétamide,
- le méthylamino-2 N-(mercapto-5 pentyl) acétamide,
- le méthylamino-2 N-(mercapto-2 éthoxy)-2′éthyl acétamide,
- l'éthylamino-2 N-(mercapto-2 éthyl) acétamide,
- l'éthylamino-2 N-(mercapto-3 propyl) acétamide,
- l'éthylamino-2 N-(mercapto-5 pentyl) acétamide,
- l'éthylamino-2 N-[(mercapto-2 éthoxy)-2′éthyl] acétamide,
- l'éthylamino-2 méthylamino-2 N-(mercapto-2 éthyl) acétamide,
- le propylamino-2 N-(mercapto-2 éthyl) acétamide,
- l'isopropylamino-2 N-(mercapto-2 éthyl) acétamide,
- le butylamino-2 N-(mercapto-2 éthyl) acétamide,
- l'éthylméthylamino-2 N-(mercapto-2 éthyl) acétamide,
- le diméthylamino-3 N-(mercapto-2 éthyl) propionamide,
- le diméthylamino-3 N-(mercapto-3 propyl) propionamide,
- le diméthylamino-3 N-(mercapto-5 pentyl) propionamide,
- le diméthylamino-3 N-[(mercapto-2 éthoxy)-2′éthyl] propionamide,
- le méthylamino-3 N-(mercapto-2 éthyl) propionamide,
- le méthylamino-3 N-(mercapto-3 propyl) propionamide,
- le méthylamino-3 N-(mercapto-5 pentyl) propionamide,
- le méthylamino-3 N-[(mercapto-2 éthoxy)-2′éthyl] propionamide,
- le diméthylamino-2 N-(mercapto-2 éthyl) propionamide,
- le diméthylamino-2 N-(mercapto-3 propyl) propionamide,
- le diméthylamino-2 N-(mercapto-5 pentyl) propionamide,
- le diméthylamino-2 N-[(mercapto-2 éthoxy)-2 éthyl] propionamide,
- le méthylamino-2 N-(mercapto-2 éthyl) propionamide,
- l'éthylamino-2 N-(mercapto-2 éthyl) propionamide,
- l'isopropylamino-2 N-(mercapto-2 éthyl) propionamide,
- le méthylamino-2 N-(mercapto-2 éthyl) butanamide,
- le diméthylamino-4 N-(mercapto-2 éthyl) butanamide,
- le diméthylamino-4 N-(mercapto-3 propyl) butanamide,
- le diméthylamino-4 N-(mercapto-5 pentyl) butanamide,
- le diméthylamino-4 N-[(mercapto-2 éthoxy)-2′éthyl] butanamide,
- le méthylamino-4 N-(mercapto-2 éthyl) butanamide,
- l'éthylamino-4 N-(mercapto-2 éthyl) butanamide,
- le diméthylamino-2 méthyl-3 N-(mercapto-2 éthyl) butanamide,
- le diméthylamino-2 méthyl-4 N-(mercapto-2 éthyl) pentanamide,
- le méthylamino-2 méthyl-4 N-(mercapto-2 éthyl) pentanamide,
- le diméthylamino-2 méthyl-3 N-(mercapto-2 éthyl) pentanamide et
- le méthylamino-2 méthyl-3 N-(mercapto-2 éthyl) pentanamide.

3. Composés selon la revendication 1, caractérisés par le fait que les disulfures sont choisis parmi :
le N,N′-(dithiodiéthanediyl-2,1) bis[diméthylamino-2-acétamide],
le N,N′-(dithiodiéthanediyl-2,1) bis[diméthylamino-2 propionamide]
le N,N′-(dithiodiéthanediyl-2,1) bis[diméthylamino-3 propionamide]
le N,N′-(dithiodiéthanediyl-2,1) bis[diméthylamino-4 butyramide],
le N,N′-(dithiodiéthanediyl-2,1) bis[méthylamino-2 acétamide],
le N,N′-[dithiobis (triméthylène)] bis[diméthylamino-2 acétamide],
le N,N′-[dithiobis (pentaméthylène)] bis[diméthylamino-2 acétamide]
le N,N′-[dithiobis (éthoxy-2 éthyl)] bis[diméthylamine-2 acétamide],
le N,N′-(dithiodiéthanediyl-2,1) bis[méthylamino-3 propionamide],
le N,N′-(dithiodiéthanediyl-2,1) bis[diéthylamino-2 acétamide],
et le N,N′-(dithiodiéthanediyl-2,1) bis[éthylamino-2 acétamide].

4. Composés nouveaux caractérisés par le fait qu'ils répondent à la formule générale (II) suivante : 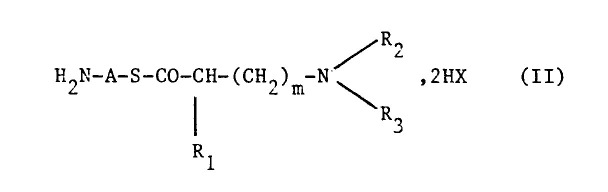 dans laquelle :
A, R₁, R₂, et R₃ et m ont les mêmes significations que celles données à la revendication 1 pour la formule générale (I), et
X est Cl ou Br.

5. Composés selon la revendication 4, caractérisé par le fait qu'ils sont choisis parmi :
- le dichlorhydrate du N,N-diméthyl thioglycinate d'amino-2 éthyle,
- le dichlorhydrate du N,N-diméthyl thioglycinate d'amino-3 propyle,
- le dichlorhydrate du N,N-diméthyl thioglycinate d'amino-5 pentyle,
- le dichlorhydrate du N,N-diméthyl thioglycinate d'(amino-2 éthoxy)-2′éthyle,
- le dichlorhydrate du N,N-diéthyl thioglycinate d'amino-2 éthyle,
- le dichlorhydrate du N,N-diéthyl thioglycinate d'amino-3 propyle,
- le dichlorhydrate du N,N-diéthyl thioglycinate d'amimo-5 pentyle,
- le dichlorhydrate du N,N-diéthyl thioglycinate d'(amino-2 éthoxy)-2′éthyle,
- le dichlorhydrate du N-méthyl thioglycinate d'amino-2 éthyle,
- le dichlorhydrate du N-méthyl thioglycinate d'amino-3 propyle,
- le dichlorhydrate du N-méthyl thioglycinate d'amino-5 pentyle,
- le dichlorhydrate du N-méthyl thioglycinate d'(amino-2 éthoxy)-2′éthyle,
- le dichlorhydrate du N-éthyl thioglycinate d'amino-2 éthyle,
- le dichlorhydrate du N-éthyl thioglycinate d'amino-3 propyle,
- le dichlorhydrate du N-éthyl thioglycinate d'amino-5 pentyle,
- le dichlorhydrate du N-éthyl thioglycinate d'(amino-2 éthoxy)-2′ éthyle,
- le dichlorhydrate du N-éthyl N-méthyl thioglycinate d'amino-2 éthyle,
- le dichlorhydrate du N-propyl thioglycinate d'amino-2 éthyle,
- le dichlorhydrate du N-isopropyl thioglycinate d'amino-2 éthyle,
- le dichlorhydrate du N-butyl thioglycinate d'amino-2 éthyle,
- le dichlorhydrate du diméthylamino-3 thiopropionate d'amino-2 éthyle,
- le dichlorhydrate du diméthylamino-3 thiopropionate d'amino-3 propyle,
- le dichlorhydrate du diméthylamino-3 thiopropionate d'amino-5 pentyle,
- le dichlorhydrate du diméthylamino-3 thiopropionate d'(amino-2 éthoxy)-2′éthyle,
- le dichlorhydrate du méthylamino-3 thiopropionate d'amino-2 éthyle,
- le dichlorhydrate du méthylamino-3 thiopropionate d'amino-3 propyle,
- le dichlorhydrate du méthylamino-3 thiopropionate d'amino-5 pentyle,
- le dichlorhydrate du méthylamino-3 thiopropionate d'(amino-2 éthoxy)-2′éthyle,
- le dichlorhydrate du N,N-diméthyl thioalaninate d'amino-2 éthyle,
- le dichlorhydrate du N,N-diméthyl thioalaninate d'amino-3 propyle,
- le dichlorhydrate du N,N-diméthyl thioalaninate d'amino-5 penthyle,
- le dichlorhydrate du N,N-diméthyl thioalaninate d'(amino-2 éthoxy)-2′éthyle,
- le dichlorhydrate du N-méthyl thioalaninate d'amino-2 éthyle,
- le dichlorhydrate du N-éthyl thioalaninate d'amino-2 éthyle,
- le dichlorhydrate du N-isopropyl thioalaninate d'amino-2 éthyle,
- le dichlorhydrate du méthylamino-2 thiobutyrate d'amino-2 éthyle,
- le dichlorhydrate du diméthylamino-4 thiobutyrate d'amino-2 éthyle,
- le dichlorhydrate du diméthylamino-4 thiobutyrate d'amino-3 propyle,
- le dichlorhydrate du diméthylamino-4 thiobutyrate d'amino-5 pentyle,
- le dichlorhydrate du diméthylamino-4 thiobutyrate d'(amino-2 éthoxy)-2′éthyle,
- le dichlorhydrate du méthylamino-4 thiobutyrate d'amino-2 éthyle,
- le dichlorhydrate de l'éthylamino-4 thiobutyrate d'amino-2 éthyle,
- le dichlorhydrate du N,N-diméthylamino thiovalinate d'amino-2 éthyle,
- le dichlorhydrate du N,N-diméthylamino thioleucinate d'amino-2 éthyle,
- le dichlorhydrate du N-méthylamino thioleucinate d'amino-2 éthyle,
- le dichlorhydrate du N,N-diméthylamino thioisoleucinate d'amino-2 éthyle,
- le dichlorhydrate du N-méthylamino thioisoleucinate d'amino-2 éthyle,
- le dichlorhydrate du thiopipécolinate d'amino-2 éthyle et
- le dichlorhydrate de l'hexahydro isothionicotinate d'amino-2 éthyle.

6. Procédé de préparation des alklylamino-mercaptoalkylamides de formule (I) selon la revendication 1 ou 2 caractérisé par le fait qu'il consiste à faire réagir dans un solvant inerte un aminothiol de formule H₂N-A-SH sur un halogénure d'acide d'un alkylaminoacide de formule : 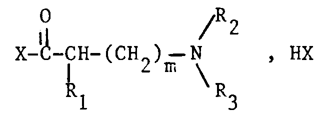 A, R₁ R₂, R₃ et m ayant les mêmes significations que celles données à la revendication 1, et X est Cl ou Br, pour obtenir un thioester de formule : 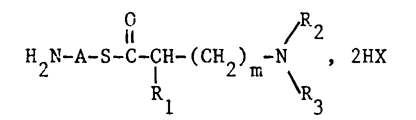 et à procéder à son réarrangement en milieu hydroalcoolique en présence d'une base.

7. Composition cosmétique pour le permier temps d'une opération de déformation permanente des cheveux caractérisée par le fait qu'elle contient, dans un véhicule cosmétique approprié, en tant qu'agent réducteur, au moins un alkylamino-mercaptoalkylamide ayant la formule générale suivante : 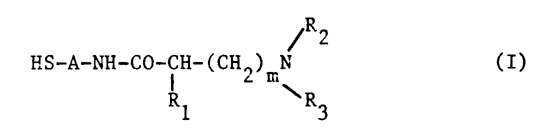 dans laquelle :
A représente le radical divalent -(CH₂)ₙ-,
n étant un nombre entier compris entre 2 et 5, ou le radical divalent -(CH₂)₂-O-(CH₂)₂-
m est égal à 0,1 ou 2
R₁ représente un atome d'hydrogène ou un radical alkyle inférieur, linéaire ou ramifié, ayant de 1 à 5 atomes de carbone,
R₂ et R₃, identiques ou différents, représentent un atome d'hydrogène ou un radical alkyle inférieur, linéaire ou ramifié, ayant de 1 à 4 atomes de carbone, R₂ et R₃ ne pouvant simultanément représenter un atome d'hydrogène,
et les sels desdits composés de formule (I).

8. Composition selon la revendication 7, caractérisée pan le fait que les sels des composés de formule (I) sont choisis parmi les chlorhydrates, bromhydrates, citrates,oxalates et acétates.

9. Composition selon la revendication 7 ou 8, caractérisée par le fait que les composés sont choisis parmi :
- le diméthylamino-2 N-(mercapto-2 éthyl) acétamide,
- le diméthylamino-2 N-(mercapto-3 propyl) acétamide,
- le diméthylamino-2 N-(mercapto-5 pentyl) acétamide,
- le diméthylamino-2 N-[(mercapto-2-éthoxy)-2′éthyl] acétamide,
- le diéthylamino-2 N-(mercapto-2 éthyl) acétamide,
- le diéthylamino-2 N-(mercapto-3 propyl) acétamide,
- le diéthylamino-2 N-(mercapto-5 pentyl) acétamide,
- le diéthylamino-2 N-[(mercapto-2 éthoxy)-2′éthyl] acétamide,
- le méthylamino-2 N-(mercapto-2 éthyl) acétamide,
- le méthylamino-2 N-(mercapto-3 propyl) acétamide,
- le méthylamino-2 N-(mercapto-5 pentyl) acétamide,
- le méthylamino-2 N-(mercapto-2 éthoxy)-2′éthyl acétamide,
- l'éthylamino-2 N-(mercapto-2 éthyl) acétamide,
- l'éthylamino-2 N-(mercapto-3 propyl) acétamide,
- l'éthylamino-2 N-(mercapto-5 pentyl) acétamide,
- l'éthylamino-2 N[(mercapto-2 éthoxy)-2′éthyl] acétamide,
- l'éthylamino-2 méthylamino-2 N-(mercapto-2 éthyl) acétamide,
- le propylamino-2 N-(mercapto-2 éthyl) acétamide,
- l'isopropylamino-2 N-(mercapto-2 éthyl) acétamide,
- le butylamino-2 N-(mercapto-2 éthyl) acétamide,
- l'éthylméthylamino-2 N-(mercapto-2 éthyl) acétamide,
- le diméthylamino-3 N-(mercapto-2 éthyl) propionamide,
- le diméthylamino-3 N-(mercapto-3 propyl) propionamide,
- le diméthylamino-3 N-(mercapto-5 pentyl) propionamide,
- le diméthylamino-3 N-[(mercapto-2 éthoxy)-2′éthyl] propionamide,
- le méthylamino-3 N-(mercapto-2 éthyl) propionamide,
- le méthylamino-3 N-(mercapto-3 propyl) propionamide,
- le méthylamino-3 N-(mercapto-5 pentyl) propionamide,
- le méthylamino-3 N-[(mercapto-2 éthoxy)-2′éthyl] propionamide,
- le diméthylamino-2 N-(mercapto-2 éthyl) propionamide,
- le diméthylamino-2 N-(mercapto-3 propyl) propionamide,
- le diméthylamino-2 N-(mercapto-5 pentyl) propionamide,
- le diméthylamino-2 N-[(mercapto-2 éthoxy)-2 éthyl] propionamide,
- le méthylamino-2 N-(mercapto-2 éthyl) propionamide,
- l'éthylamino-2 N-(mercapto-2 éthyl) propionamide,
- l'isopropylamino-2 N-(mercapto-2 éthyl) propionamide,
- le méthylamino-2 N-(mercapto-2 éthyl) butanamide,
- le diméthylamino-4 N-(mercapto-2 éthyl) butanamide,
- le diméthylamino-4 N-(mercapto-3 propyl) butanamide,
- le diméthylamino-4 N-(mercapto-5 pentyl) butanamide,
- le diméthylamino-4 N-[(mercapto-2 éthoxy)-2′éthyl] butanamide,
- le méthylamino-4 N-(mercapto-2 éthyl) butanamide,
- l'éthylamino-4 N-(mercapto-2 éthyl) butanamide,
- le diméthylamino-2 méthyl-3 N-(mercapto-2 éthyl) butanamide,
- le diméthylamino-2 méthyl-4 N-(mercapto-2 éthyl) pentanamide,
- le méthylamino-2 méthyl-4 N-(mercapto-2 éthyl) pentanamide,
- le diméthylamino-2 méthyl-3 N-(mercapto-2 éthyl) pentanamide et
- le méthylamino-2 méthyl-3 N-(mercapto-2 éthyl) pentanamide.

10. Composition selon les revendications 7 à 9, caractérisée par le fait que le composé de formule (I) est formé in situ à partir de son précurseur thioester ayant la formule générale (II) suivante : 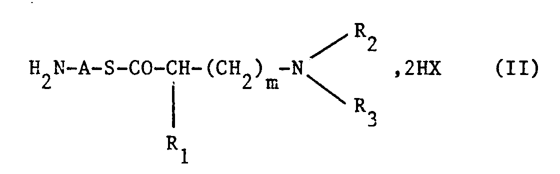 dans laquelle :
A, R₁, R₂, et R₃et m ont les mêmes significations que celles données à la revendication 1 pour la formule générale (I), et
X est Cl ou Br.

11. Composition selon l'une quelconque des revendications 7 à 10, caractérisée par le fait que le composé de formule (I) ou (II) est présent à une concentration comprise entre 2 et 30 % et de préférence entre 5 et 25 % en poids par rapport au poids total de la composition.

12. Composition selon l'une quelconque des revendications 7 à 11, caractérisée par le fait qu'elle présente un pH compris entre 4,5 et 11, et de préférence entre 6 et 10, obtenu à l'aide d'un agent alcalin choisi parmi l'ammoniaque, la monoéthanolamine, la diéthanolamine, la triéthanolamine, un carbonate ou un bicarbonate alcalin ou d'ammonium, un hydroxyde alcalin ou à l'aide d'un agent acidifiant choisi parmi l'acide chlorhydrique, l'acide acétique, l'acide lactique, l'acide oxalique ou l'acide borique.

13. Composition selon l'une quelconque des revendications 7 à 12, caractérisée par le fait qu'elle contient en outre au moins un autre agent réducteur choisi parmi : l'acide thioglycolique, le monothioglycolate de glycérol ou de glycol, la cystéamine et ses dérivés acylés C₁-C₄, la cystéine, la N-acétylcystéine, les N-mercaptoalkylamides de sucre, l'acide β-mercaptopropionique et ses dérivés, l'acide thiolactique, l'acide thiomalique, la pantéthéine, le thioglycérol, un sulfite ou un bisulfite d'un métal alcalin ou alcalino-terreux, les N-(mercaptoalkyl)ω -hydroxyalkylamides, les N-mono ou N,N-dialkyl mercapto 4-butyramides, les aminomercaptoalkylamides et les dérivés des acides N-(mercaptoalkyl) succinamiques ou des N-(mercaptoalkyl) succinimides.

14. Composition selon l'une quelconque des revendications 7 à 13, caractérisée par le fait qu'elle contient, en outre, au moins un polymère cationique, un agent adoucissant, un hydrolysat de protéines, une cire, un agent opacifiant, un parfum, un colorant, un agent tensio-actif non-ionique, un alcool, un agent traitant ou encore un agent de pénétration.

15. Composition selon l'une quelconque des revendications 7 à 14, caractérisée par le fait qu'elle contient, en outre, au moins un disulfure, la composition étant du type auto-neutralisante.

16. Composition selon la revendication 15, caractérisée par le fait que le disulfure est l'acide dithioglycolique, le dithioglycérol, la cystamine, la N,N′-diacétyl-cystamine, la cystine, la panthétine ou un disulfure de N-(mercaptoalkyl)ω-hydroxy alkylamide ou de N-mono ou N,N-dialkyl mercapto 4-butyramide, un disulfure d'aminomercaptoalkylamide, un disulfure des dérivés des acides N-(mercaptoalkyl) succinamiques ou des N-(mercaptoalkyl) succinimides.

17. Composition selon la revendication 15, caractérisée par le fait que le disulfure correspond à la formule générale (III) suivante 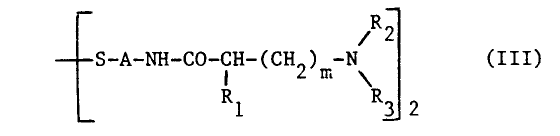 dans laquelle :
A, R₁, R₂ et R₃ et m ont les mêmes significations que celle donnée à la revendication 1 pour la formule générale (I) à l'exclusion du composé de formule (III) dans laquelle :
A = -(CH₂)₂-, m = O,
R₁ et R₂ = H et R₃ = -C₂H₅

18. Composition selon la revendication 17, caractérisée par de fait que le disulfure de formule (III) est choisi parmi :
le N,N′-(dithiodiéthanediyl-2,1) bis [diméthylamino-2 acétamide],
le N,N′-(dithiodiéthanediyl-2,1) bis [diméthylamino-2 propionamide]
le N,N′-(dithiodiéthanediyl-2,1) bis [diméthylamino-3 propionamide]
le N,N′-(dithiodiéthanediyl-2,1) bis [diméthylamino-4 butyramide],
le N,N′-(dithiodiéthanediyl-2,1) bis [méthylamino-2 acétamide],
le N,N′-[dithiobis (triméthylène)]bis [diméthylamino-2 acétamide],
le N,N′-[dithiobis (pentaméthylène)] bis[diméthylamino-2 acétamide],
le N,N′-[dithiobis (éthoxy-2 éthyl)] bis[diméthylamine-2 acétamide],
le N,N′-(dithiodiéthanediyl-2,1) bis [méthylamino-3 propionamide],
le N,N′-(dithiodiéthanediyl-2,1) bis [diéthylamino-2 acétamide],
et le N,N′-(dithiodiéthanediyl-2,1) bis [éthylamino-2 acétamide].

19. Composition selon l'une quelconque des revendications 15 à 18, caractérisée par le fait que le disulfure est présent en une proportion molaire par rapport au composé de formule (I) allant de 0,5 à 2,5 et de préférence de 1 à 2.

20. Procédé de déformation permanente des cheveux consistant, dans une première étape, à réduire les liaisons disulfures de la kératine par application d'une composition réductrice, puis dans une seconde étape, à reformer lesdites liaisons par application d'une composition oxydante, caractérisé par le fait que l'étape de réduction est réalisée à l'aide d'une composition cosmétique réductrice telle que revendiquée selon l'une quelconque des revendications 7 à 14.

21. Procédé selon la revendication 20, caractérisé par le fait que l'on laisse agir la composition réductrice pendant un temps compris entre 5 et 60 minutes.

## Revendications (Revendications pour l'(les) Etat(s) contractant(s) suivant(s): ES, GR)

1. Procédé de préparation d' alklylamino-mercaptoalkylamides de formule (I) suivante : 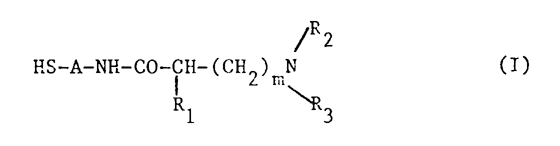 dans laquelle :
A représente le radical divalent -(CH₂)ₙ-,
n étant un nombre entier compris entre 2 et 5, ou le radical divalent -(CH₂)₂-O-(CH₂)₂-
m est égal à 0,1 ou 2
R₁ représente un atome d'hydrogène ou un radical alkyle inférieur, linéaire ou ramifié, ayant de 1 à 5 atomes de carbone,
R₂ et R₃, identiques ou différents, représentent un atome d'hydrogène ou un radical alkyle inférieur, linéaire ou ramifié, ayant de 1 à 4 atomes de carbone, R₂ et R₃ ne pouvant simultanément représenter un atome d'hydrogène, les sels desdits composés de formule (I) ainsi que leurs disulfures correspondants à l'exclusion du disulfure dans lequel :
A = -(CH₂)₂-, m = 0
R₁ et R₂ = H et R₃ = -C₂H₅,
caractérisé par le fait qu'il consiste à faire réagir dans un solvant inerte un aminothiol de formule H₂N-A-SH sur un halogénure d'acide d'un alkylaminoacide de formule : 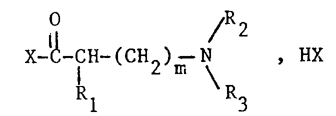 A, R₁ R₂, R₃ et m ayant les mêmes significations que celles données à la revendication 1, et X est Cl ou Br, pour obtenir un thioester de formule : 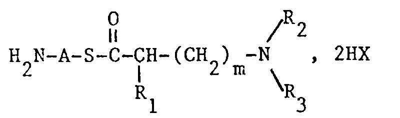 et à procéder à son réarrangement en milieu hydroalcoolique en présence d'une base.

2. Composition cosmétique pour le permier temps d'une opération de déformation permanente des cheveux caractérisée par le fait qu'elle contient, dans un véhicule cosmétique approprié, en tant qu'agent réducteur, au moins un alkylamino-mercaptoalkylamide de formule (I) suivante : 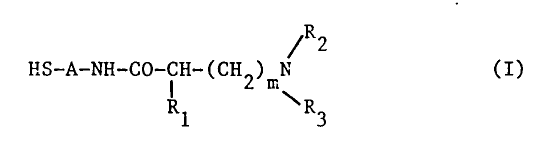 dans laquelle :
A représente le radical divalent -(CH₂)ₙ-,
n étant un nombre entier compris entre 2 et 5, ou le radical divalent -(CH₂)₂-O-(CH₂)₂-
m est égal à 0,1 ou 2
R₁ représente un atome d'hydrogène ou un radical alkyle inférieur, linéaire ou ramifié, ayant de 1 à 5 atomes de carbone,
R₂ et R₃, identiques ou différents, représentent un atome d'hydrogène ou un radical alkyle inférieur, linéaire ou ramifié, ayant de 1 à 4 atomes de carbone, R₂ et R₃ ne pouvant simultanément représenter un atome d'hydrogène,
et les sels desdits composés de formule (I).

3. Composition selon la revendication 2, caractérisée par le fait que les sels des composés de formule (I) sont choisis parmi les chlorhydrates, bromhydrates, citrates, oxalates et acétates.

4. Composition selon la revendication 2 ou 3, caractérisée par le fait que les composés sont choisis parmi :
- le diméthylamino-2 N-(mercapto-2 éthyl) acétamide,
- le diméthylamino-2 N-(mercapto-3 propyl) acétamide,
- le diméthylamino-2 N-(mercapto-5 pentyl) acétamide,
- le diméthylamino-2 N-[(mercapto-2-éthoxy)-2'éthyl] acétamide,
- le diéthylamino-2 N-(mercapto-2 éthyl) acétamide,
- le diéthylamino-2 N-(mercapto-3 propyl) acétamide,
- le diéthylamino-2 N-(mercapto-5 pentyl) acétamide,
- le diéthylamino-2 N-[(mercapto-2 éthoxy)-2'éthyl] acétamide,
- le méthylamino-2 N-(mercapto-2 éthyl) acétamide,
- le méthylamino-2 N-(mercapto-3 propyl) acétamide,
- le méthylamino-2 N-(mercapto-5 pentyl) acétamide,
- le méthylamino-2 N-(mercapto-2 éthoxy)-2'éthyl acétamide,
- l'éthylamino-2 N-(mercapto-2 éthyl) acétamide,
- l'éthylamino-2 N-(mercapto-3 propyl) acétamide,
- l'éthylamino-2 N-(mercapto-5 pentyl) acétamide,
- l'éthylamino-2 N-[(mercapto-2 éthoxy)-2'éthyl] acétamide,
- l'éthylamino-2 méthylamino-2 N-(mercapto-2 éthyl) acétamide,
- le propylamino-2 N-(mercapto-2 éthyl) acétamide,
- l'isopropylamino-2 N-(mercapto-2 éthyl) acétamide,
- le butylamino-2 N-(mercapto-2 éthyl) acétamide,
- l'éthylméthylamino-2 N-(mercapto-2 éthyl) acétamide,
- le diméthylamino-3 N-(mercapto-2 éthyl) propionamide,
- le diméthylamino-3 N-(mercapto-3 propyl) propionamide,
- le diméthylamino-3 N-(mercapto-5 pentyl) propionamide,
- le diméthylamino-3 N-[(mercapto-2 éthoxy)-2'éthyl] propionamide,
- le méthylamino-3 N-(mercapto-2 éthyl) propionamide,
- le méthylamino-3 N-(mercapto-3 propyl) propionamide,
- le méthylamino-3 N-(mercapto-5 pentyl) propionamide,
- le méthylamino-3 N-[(mercapto-2 éthoxy)-2'éthyl] propionamide,
- le diméthylamino-2 N-(mercapto-2 éthyl) propionamide,
- le diméthylamino-2 N-(mercapto-3 propyl) propionamide,
- le diméthylamino-2 N-(mercapto-5 pentyl) propionamide,
- le diméthylamino-2 N-[(mercapto-2 éthoxy)-2 éthyl] propionamide,
- le méthylamino-2 N-(mercapto-2 éthyl) propionamide,
- l'éthylamino-2 N-(mercapto-2 éthyl) propionamide,
- l'isopropylamino-2 N-(mercapto-2 éthyl) propionamide,
- le méthylamino-2 N-(mercapto-2 éthyl) butanamide,
- le diméthylamino-4 N-(mercapto-2 éthyl) butanamide,
- le diméthylamino-4 N-(mercapto-3 propyl) butanamide,
- le diméthylamino-4 N-(mercapto-5 pentyl) butanamide,
- le diméthylamino-4 N-[(mercapto-2 éthoxy)-2'éthyl] butanamide,
- le méthylamino-4 N-(mercapto-2 éthyl) butanamide,
- l'éthylamino-4 N-(mercapto-2 éthyl) butanamide,
- le diméthylamino-2 méthyl-3 N-(mercapto-2 éthyl) butanamide,
- le diméthylamino-2 méthyl-4 N-(mercapto-2 éthyl) pentanamide,
- le méthylamino-2 méthyl-4 N-(mercapto-2 éthyl) pentanamide,
- le diméthylamino-2 méthyl-3 N-(mercapto-2 éthyl) pentanamide et
- le méthylamino-2 méthyl-3 N-(mercapto-2 éthyl) pentanamide.

5. Composition selon les revendications 2 à 4, caractérisée par le fait que le composé de formule (I) est formé in situ à partir de son précurseur thioester ayant la formule générale (II) suivante : 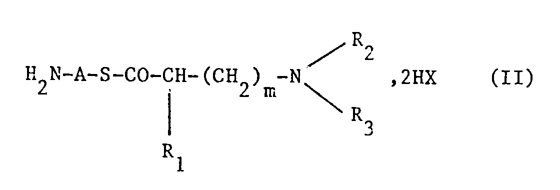 dans laquelle :
A, R₁, R₂, et R₃et m ont les mêmes significations que celles données à la revendication 1 pour la formule générale (I), et
X est Cl ou Br.

6. Composition selon l'une quelconque des revendications 2 à 5, caractérisée par le fait que le composé de formule (I) ou (II) est présent à une concentration comprise entre 2 et 30 % et de préférence entre 5 et 25 % en poids par rapport au poids total de la composition.

7. Composition selon l'une quelconque des revendications 2 à 6, caractérisée par le fait qu'elle présente un pH compris entre 4,5 et 11, et de préférence entre 6 et 10, obtenu à l'aide d'un agent alcalin choisi parmi l'ammoniaque, la monoéthanolamine, la diéthanolamine, la triéthanolamine, un carbonate ou un bicarbonate alcalin ou d'ammonium, un hydroxyde alcalin ou à l'aide d'un agent acidifiant choisi parmi l'acide chlorhydrique, l'acide acétique, l'acide lactique, l'acide oxalique ou l'acide borique.

8. Composition selon l'une quelconque des revendications 2 à 7, caractérisée par le fait qu'elle contient en outre au moins un autre agent réducteur choisi parmi : l'acide thioglycolique, le monothioglycolate de glycérol ou de glycol, la cystéamine et ses dérivés acylés C₁-C₄, la cystéine, la N-acétylcystéine, les N-mercaptoalkylamides de sucre, l'acide β-mercaptopropionique et ses dérivés, l'acide thiolactique, l'acide thiomalique, la pantéthéine, le thioglycérol, un sulfite ou un bisulfite d'un métal alcalin ou alcalino-terreux, les N-(mercaptoalkyl)ω -hydroxyalkylamides, les N-mono ou N,N-dialkyl mercapto 4-butyramides, les aminomercaptoalkylamides et les dérivés des acides N-(mercaptoalkyl) succinamiques ou des N-(mercaptoalkyl) succinimides.

9. Composition selon l'une quelconque des revendications 2 à 8, caractérisée par le fait qu'elle contient, en outre, au moins un polymère cationique, un agent adoucissant, un hydrolysat de protéines, une cire, un agent opacifiant, un parfum, un colorant, un agent tensio-actif non-ionique, un alcool, un agent traitant ou encore un agent de pénétration.

10. Composition selon l'une quelconque des revendications 2 à 9, caractérisée par le fait qu'elle contient, en outre, au moins un disulfure, la composition étant du type auto-neutralisante.

11. Composition selon la revendication 10, caractérisée par le fait que le disulfure est l'acide dithioglycolique, le dithioglycérol, la cystamine, la N,N'-diacétyl-cystamine, la cystine, la panthétine ou un disulfure de N-(mercaptoalkyl)ω-hydroxy alkylamide ou de N-mono ou N,N-dialkyl mercapto 4-butyramide, un disulfure d'aminomercaptoalkylamide, un disulfure des dérivés des acides N-(mercaptoalkyl) succinamiques ou des N-(mercaptoalkyl) succinimides.

12. Composition selon la revendication 11, caractérisée par le fait que le disulfure correspond à la formule générale (III) suivante 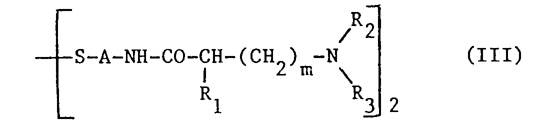 dans laquelle :
A, R₁, R₂ et R₃ et m ont les mêmes significations que celle donnée à la revendication 1 pour la formule générale (I) à l'exclusion du composé de formule (III) dans laquelle :
A = -(CH₂₎₂-, m = O,
R₁ et R₂ = H et R₃ = -C₂H₅

13. Composition selon la revendication 12, caractérisée par le fait que le disulfure de formule (III) est choisi parmi :
le N,N'-(dithiodiéthanediyl-2,1) bis [diméthylamino-2 acétamide],
le N,N'-(dithiodiéthanediyl-2,1) bis [diméthylamino-2 propionamide]
le N,N'-(dithiodiéthanediyl-2,1) bis [diméthylamino-3 propionamide]
le N,N'-(dithiodiéthanediyl-2,1) bis [diméthylamino-4 butyramide],
le N,N'-(dithiodiéthanediyl-2,1) bis [méthylamino-2 acétamide],
le N,N'-[dithiobis (triméthylène)] bis [diméthylamino-2 acétamide],
le N,N'-[dithiobis (pentaméthylène)] bis [diméthylamino-2 acétamide],
le N,N'-[dithiobis (éthoxy-2 éthyl)] bis [diméthylamine-2 acétamide],
le N,N'-(dithiodiéthanediyl-2,1) bis [méthylamino-3 propionamide],
le N,N'-(dithiodiéthanediyl-2,1) bis [diéthylamino-2 acétamide],
et le N,N'-(dithiodiéthanediyl-2,1) bis [éthylamino-2 acétamide].

14. Composition selon l'une quelconque des revendications 10 à 13, caractérisée par le fait que le disulfure est présent en une proportion molaire par rapport au composé de formule (I) allant de 0,5 à 2,5 et de préférence de 1 à 2.

15. Procédé de déformation permanente des cheveux consistant, dans une première étape, à réduire les liaisons disulfures de la kératine par application d'une composition réductrice, puis dans une seconde étape, à reformer lesdites liaisons par application d'une composition oxydante, caractérisé par le fait que l'étape de réduction est réalisée à l'aide d'une composition cosmétique réductrice telle que revendiquée selon l'une quelconque des revendications 2 à 9.

16. Procédé selon la revendication 15, caractérisé par le fait que l'on laisse agir la composition réductrice pendant un temps compris entre 5 et 60 minutes.

## Claims (Claims for the following Contracting State(s): AT, BE, CH, DE, DK, FR, GB, IT, LI, NL, PT, SE)

1. Novel compounds, characterized in that they correspond to the following general formula (I): 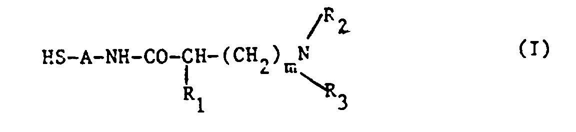 in which:
A represents the divalent radical -(CH₂)ₙ-,
n being an integer between 2 and 5, or the divalent radical -(CH₂)₂-O-(CH₂)₂-
m is equal to 0, 1 or 2
R₁ represents a hydrogen atom or a linear or branched lower alkyl radical having from 1 to 5 carbon atoms,
R₂ and R₃, which are identical or different, represent a hydrogen atom or a linear or branched lower alkyl radical having from 1 to 4 carbon atoms, it not being possible for R₂ and R₃ simultaneously to represent a hydrogen atom,
the salts of the said compounds of formula (I) and their corresponding disulphides, with the exception of the disulphide in which:
A = -(CH₂)₂-, m = 0
R₁ and R₂ = H and R₃ = -C₂H₅.

2. Compounds according to Claim 1, characterized in that they are chosen from :
- 2-dimethylamino-N-(2-mercaptoethyl)acetamide,
- 2-dimethylamino-N-(3-mercaptopropyl)acetamide,
- 2-dimethylamino-N-(5-mercaptopentyl)acetamide,
- 2-dimethylamino-N-[2'-(2-mercaptoethoxy)ethyl]acetamide,
- 2-diethylamino-N-(2-mercaptoethyl)acetamide,
- 2-diethylamino-N-(3-mercaptopropyl)acetamide,
- 2-diethylamino-N-(5-mercaptopentyl)acetamide,
- 2-diethylamino-N-[2'-(2-mercaptoethoxy)ethyl]acetamide,
- 2-methylamino-N-(2-mercaptoethyl)acetamide,
- 2-methylamino-N-(3-mercaptopropyl)acetamide,
- 2-methylamino-N-(5-mercaptopentyl)acetamide,
- 2-methylamino-N-[2'-(2-mercaptoethoxy)ethyl]acetamide,
- 2-ethylamino-N-(2-mercaptoethyl)acetamide,
- 2-ethylamino-N-(3-mercaptopropyl)acetamide,
- 2-ethylamino-N-(5-mercaptopentyl)acetamide,
- 2-ethylamino-N-[2'-(2-mercaptoethoxy)ethyl]acetamide,
- 2-ethylamino-2-methylamino-N-(2-mercaptoethyl)acetamide,
- 2-propylamino-N-(2-mercaptoethyl)acetamide,
- 2-isopropylamino-N-(2-mercaptoethyl)acetamide,
- 2-butylamino-N-(2-mercaptoethyl)acetamide,
- 2-ethylmethylamino-N-(2-mercaptoethyl)acetamide,
- 3-dimethylamino-N-(2-mercaptoethyl)propionamide,
- 3-dimethylamino-N-(3-mercaptopropyl)propionamide,
- 3-dimethylamino-N-(5-mercaptopentyl)propionamide,
- 3-dimethylamino-N-[2'-(2-mercaptoethoxy)ethyl]propionamide
- 3-methylamino-N-(2-mercaptoethyl)propionamide,
- 3-methylamino-N-(3-mercaptopropyl)propionamide,
- 3-methylamino-N-(5-mercaptopentyl)propionamide,
- 3-methylamino-N-[2'-(2-mercaptoethoxy)ethyl]propionamide,
- 2-dimethylamino-N-(2-mercaptoethyl)propionamide,
- 2-dimethylamino-N-(3-mercaptopropyl)propionamide,
- 2-dimethylamino-N-(5-mercaptopentyl)propionamide,
- 2-dimethylamino-N-[2'-(2-mercaptoethoxy)ethyl]propionamide,
- 2-methylamino-N-(2-mercaptoethyl)propionamide,
- 2-ethylamino-N-(2-mercaptoethyl)propionamide,
- 2-isopropylamino-N-(2-mercaptoethyl)propionamide,
- 2-methylamino-N-(2-mercaptoethyl)butanamide,
- 4-dimethylamino-N-(2-mercaptoethyl)butanamide,
- 4-dimethylamino-N-(3-mercaptopropyl)butanamide,
- 4-dimethylamino-N-(5-mercaptopentyl)butanamide,
- 4-dimethylamino-N-[2'-(2-mercaptoethoxy)ethyl]butanamide,
- 4-methylamino-N-(2-mercaptoethyl)butanamide,
- 4-ethylamino-N-(2-mercaptoethyl)butanamide,
- 2-dimethylamino-3-methyl-N-(2-mercaptoethyl)butanamide,
- 2-dimethylamino-4-methyl-N-(2-mercaptoethyl)pentanamide,
- 2-methylamino-4-methyl-N-(2-mercaptoethyl)pentanamide,
- 2-dimethylamino-3-methyl-N-(2-mercaptoethyl)pentanamide, and
- 2-methylamino-3-methyl-N-(2-mercaptoethyl)pentanamide.

3. Compounds according to Claim 1, characterized in that the disulphides are chosen from:
N,N'-(dithiodi(2,1-ethanediyl))bis[2-dimethylaminoacetamide],
N,N'-(dithiodi(2,1-ethanediyl))bis[2-dimethylaminopropionamide],
N,N'-(dithiodi(2,1-ethanediyl))bis[3-dimethylaminopropionamide],
N,N'-(dithiodi(2,1-ethanediyl))bis[4-dimethylaminobutyramide],
N,N'-(dithiodi(2,1-ethanediyl))bis[2-methylaminoacetamide],
N,N'-[dithiobis(trimethylene)]bis[2-dimethylaminoacetamide],
N,N'-[dithiobis(pentamethylene)]bis[2-dimethylaminoacetamide],
N,N'-[dithiobis(2-ethoxyethyl)]bis[2-dimethylaminoacetamide],
N,N'-(dithiodi(2,1-ethanediyl))bis[3-methylaminopropionamide],
N,N'-(dithiodi(2,1-ethanediyl))bis[2-diethylaminoacetamide],
and N,N'-(dithiodi(2,1-ethanediyl))bis[2-ethylaminoacetamide].

4. Novel compounds, characterized in that they correspond to the following general formula (II): 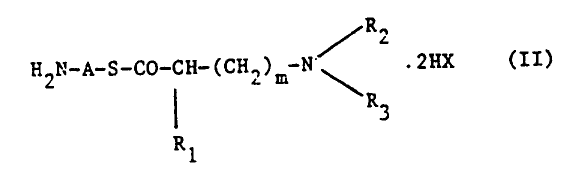 in which:
A, R₁, R₂, R₃ and m have the same meanings as those given in Claim 1 for the general formula (I), and
X is Cl or Br.

5. Compounds according to Claim 4, characterized in that they are chosen from:
- 2-aminoethyl N,N-dimethylthioglycinate dihydrochloride,
- 3-aminopropyl N,N-dimethylthioglycinate dihydrochloride,
- 5-aminopentyl N,N-dimethylthioglycinate dihydrochloride,
- 2'-(2-aminoethoxy)ethyl N,N-dimethylthioglycinate dihydrochloride,
- 2-aminoethyl N,N-diethylthioglycinate dihydrochloride,
- 3-aminopropyl N,N-diethylthioglycinate dihydrochloride,
- 5-aminopentyl N,N-diethylthioglycinate dihydrochloride,
- 2'-(2-aminoethoxy)ethyl N,N-diethylthioglycinate dihydrochloride,
- 2-aminoethyl N-methylthioglycinate dihydrochloride,
- 3-aminopropyl N-methylthioglycinate dihydrochloride,
- 5-aminopentyl N-methylthioglycinate dihydrochloride,
- 2'-(2-aminoethoxy)ethyl N-methylthioglycinate dihydrochloride,
- 2-aminoethyl N-ethylthioglycinate dihydrochloride,
- 3-aminopropyl N-ethylthioglycinate dihydrochloride,
- 5-aminopentyl N-ethylthioglycinate dihydrochloride,
- 2'-(2-aminoethoxy)ethyl N-ethylthioglycinate dihydrochloride,
- 2-aminoethyl N-ethyl-N-methylthioglycinate dihydrochloride,
- 2-aminoethyl N-propylthioglycinate dihydrochloride,
- 2-aminoethyl N-isopropylthioglycinate dihydrochloride,
- 2-aminoethyl N-butylthioglycinate dihydrochloride,
- 2-aminoethyl 3-(dimethylamino)thiopropionate dihydrochloride,
- 3-aminopropyl 3-(dimethylamino)thiopropionate dihydrochloride,
- 5-aminopentyl 3-(dimethylamino)thiopropionate dihydrochloride,
- 2'-(2-aminoethoxy)ethyl 3-(dimethylamino)thiopropionate dihydrochloride,
- 2-aminoethyl 3-(methylamino)thiopropionate dihydrochloride,
- 3-aminopropyl 3-(methylamino)thiopropionate dihydrochloride,
- 5-aminopentyl 3-(methylamino)thiopropionate dihydrochloride,
- 2'-(2-aminoethoxy)ethyl 3-(methylamino)thiopropionate dihydrochloride,
- 2-aminoethyl N,N-dimethylthioalaninate dihydrochloride,
- 3-aminopropyl N,N-dimethylthioalaninate dihydrochloride,
- 5-aminopentyl N,N-dimethylthioalaninate dihydrochloride,
- 2'-(2-aminoethoxy)ethyl N,N-dimethylthioalaninate dihydrochloride,
- 2-aminoethyl N-methylthioalaninate dihydrochloride,
- 2-aminoethyl N-ethylthioalaninate dihydrochloride,
- 2-aminoethyl N-isopropylthioalaninate dihydrochloride,
- 2-aminoethyl 2-(methylamino)thiobutyrate dihydrochloride,
- 2-aminoethyl 4-(dimethylamino)thiobutyrate dihydrochloride,
- 3-aminopropyl 4-(dimethylamino)thiobutyrate dihydrochloride,
- 5-aminopentyl 4-(dimethylamino)thiobutyrate dihydrochloride,
- 2'-(2-aminoethoxy)ethyl 4-(dimethylamino)thiobutyrate dihydrochloride,
- 2-aminoethyl 4-(methylamino)thiobutyrate dihydrochloride,
- 2-aminoethyl 4-(ethylamino)thiobutyrate dihydrochloride,
- 2-aminoethyl N,N-dimethylthiovalinate dihydrochloride,
- 2-aminoethyl N,N-dimethylthioleucinate dihydrochloride,
- 2-aminoethyl N-methylthioleucinate dihydrochloride,
- 2-aminoethyl N,N-dimethylthioisoleucinate dihydrochloride,
- 2-aminoethyl N-methylthioisoleucinate dihydrochloride,
- 2-aminoethyl thiopipecolinate dihydrochloride and
- 2-aminoethyl hexahydroisothionicotinate dihydrochloride.

6. Process for the preparation of the alkylamino mercaptoalkylamides of formula (I) according to Claim 1 or 2, characterized in that it consists in reacting, in an inert solvent, an aminothiol of formula H₂N-A-SH with an acid halide of an alkylamino acid of formula: 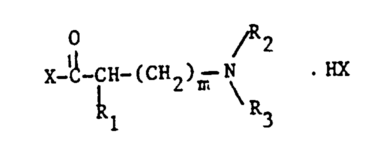 A, R₁, R₂, R₃ and m having the same meanings as those given in Claim 1, and X is Cl or Br, in order to obtain a thioester of formula: 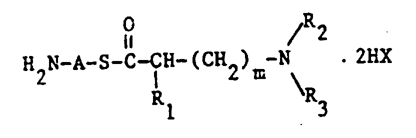 and in carrying out its rearrangement in aqueous/alcoholic medium in the presence of a base.

7. Cosmetic composition for the first step in an operation for the permanent deformation of hair, characterized in that it contains, in a suitable cosmetic vehicle, as reducing agent, at least one alkylamino mercaptoalkylamide having the following general formula: 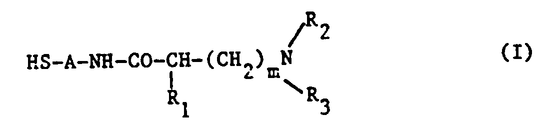 in which:
A represents the divalent radical -(CH₂)ₙ-,
n being an integer between 2 and 5, or the divalent radical -(CH₂)₂-O-(CH₂)₂-
m is equal to 0, 1 or 2
R₁ represents a hydrogen atom or a linear or branched lower alkyl radical having from 1 to 5 carbon atoms,
R₂ and R₃, which are identical or different, represent a hydrogen atom or a linear or branched lower alkyl radical having from 1 to 4 carbon atoms, it not being possible for R₂ and R₃ simultaneously to represent a hydrogen atom,
and the salts of the said compounds of formula (I).

8. Composition according to Claim 7, characterized in that the salts of the compounds of formula (I) are chosen from the hydrochlorides, hydrobromides, citrates, oxalates and acetates.

9. Composition according to Claim 7 or 8, characterized in that the compounds are chosen from:
- 2-dimethylamino-N-(2-mercaptoethyl)acetamide,
- 2-dimethylamino-N-(3-mercaptopropyl)acetamide,
- 2-dimethylamino-N-(5-mercaptopentyl)acetamide,
- 2-dimethylamino-N-[2'-(2-mercaptoethoxy)ethyl]acetamide,
- 2-diethylamino-N-(2-mercaptoethyl)acetamide,
- 2-diethylamino-N-(3-mercaptopropyl)acetamide,
- 2-diethylamino-N-(5-mercaptopentyl)acetamide,
- 2-diethylamino-N-[2'-(2-mercaptoethoxy)ethyl]acetamide,
- 2-methylamino-N-(2-mercaptoethyl)acetamide,
- 2-methylamino-N-(3-mercaptopropyl)acetamide,
- 2-methylamino-N-(5-mercaptopentyl)acetamide,
- 2-methylamino-N-[2'-(2-mercaptoethoxy)ethyl]acetamide,
- 2-ethylamino-N-(2-mercaptoethyl)acetamide,
- 2-ethylamino-N-(3-mercaptopropyl)acetamide,
- 2-ethylamino-N-(5-mercaptopentyl)acetamide,
- 2-ethylamino-N-[2'-(2-mercaptoethoxy)ethyl]acetamide,
- 2-ethylamino-2-methylamino-N-(2-mercaptoethyl)acetamide,
- 2-propylamino-N-(2-mercaptoethyl)acetamide,
- 2-isopropylamino-N-(2-mercaptoethyl)acetamide,
- 2-butylamino-N-(2-mercaptoethyl)acetamide,
- 2-ethylmethylamino-N-(2-mercaptoethyl)acetamide,
- 3-dimethylamino-N-(2-mercaptoethyl)propionamide,
- 3-dimethylamino-N-(3-mercaptopropyl)propionamide,
- 3-dimethylamino-N-(5-mercaptopentyl)propionamide,
- 3-dimethylamino-N-[2'-(2-mercaptoethoxy)ethyl]propionamide
- 3-methylamino-N-(2-mercaptoethyl)propionamide,
- 3-methylamino-N-(3-mercaptopropyl)propionamide,
- 3-methylamino-N-(5-mercaptopentyl)propionamide,
- 3-methylamino-N-[2'-(2-mercaptoethoxy)ethyl]propionamide,
- 2-dimethylamino-N-(2-mercaptoethyl)propionamide,
- 2-dimethylamino-N-(3-mercaptopropyl)propionamide,
- 2-dimethylamino-N-(5-mercaptopentyl)propionamide,
- 2-dimethylamino-N-[2'-(2-mercaptoethoxy)ethyl]propionamide,
- 2-methylamino-N-(2-mercaptoethyl)propionamide,
- 2-ethylamino-N-(2-mercaptoethyl)propionamide,
- 2-isopropylamino-N-(2-mercaptoethyl)propionamide,
- 2-methylamino-N-(2-mercaptoethyl)butanamide,
- 4-dimethylamino-N-(2-mercaptoethyl)butanamide,
- 4-dimethylamino-N-(3-mercaptopropyl)butanamide,
- 4-dimethylamino-N-(5-mercaptopentyl)butanamide,
- 4-dimethylamino-N-[2'-(2-mercaptoethoxy)ethyl]butanamide,
- 4-methylamino-N-(2-mercaptoethyl)butanamide,
- 4-ethylamino-N-(2-mercaptoethyl)butanamide,
- 2-dimethylamino-3-methyl-N-(2-mercaptoethyl)butanamide,
- 2-dimethylamino-4-methyl-N-(2-mercaptoethyl)pentanamide,
- 2-methylamino-4-methyl-N-(2-mercaptoethyl)pentanamide,
- 2-dimethylamino-3-methyl-N-(2-mercaptoethyl)pentanamide, and
- 2-methylamino-3-methyl-N-(2-mercaptoethyl)pentanamide.

10. Composition according to Claims 7 to 9, characterized in that the compound of formula (I) is formed in situ from its thioester precursor having the following general formula (II): 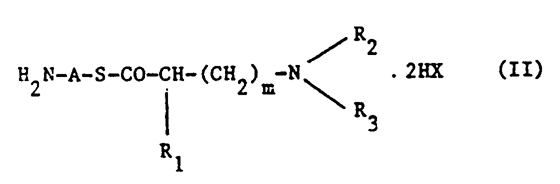 in which:
A, R₁, R₂, R₃ and m have the same meanings as those given in Claim 1 for the general formula (I), and
X is Cl or Br.

11. Composition according to any one of Claims 7 to 10, characterized in that the compound of formula (I) or (II) is present at a concentration of between 2 and 30 % and preferably between 5 and 25 % by weight with respect to the total weight of the composition.

12. Composition according to any one of Claims 7 to 11, characterized in that it has a pH of between 4.5 and 11, and preferably between 6 and 10, obtained using an alkaline agent chosen from ammonia, monoethanolamine, diethanolamine, triethanolamine, an alkali metal or ammonium carbonate or bicarbonate, or an alkali metal hydroxide or using an acidifying agent chosen from hydrochloric acid, acetic acid, lactic acid, oxalic acid or boric acid.

13. Composition according to any one of Claims 7 to 12, characterized in that it additionally contains at least one other reducing agent chosen from: thioglycolic acid, glycerol or glycol monothioglycolate, cysteamine and its C₁-C₄ acylated derivatives, cysteine, N-acetylcysteine, N-mercaptoalkylamides of sugar, β-mercaptopropionic acid and its derivatives, thiolactic acid, thiomalic acid, pantetheine, thioglycerol, an alkali metal or alkaline-earth metal sulphite or bisulphite, N-mercaptoalkyl-ω-hydroxyalkylamides, N-mono- or N,N-dialkyl-4-mercaptobutyramides, aminomercaptoalkylamides and derivatives of N-(mercaptoalkyl)succinamic acids or of N-(mercaptoalkyl)succinimides.

14. Composition according to any one of Claims 7 to 13, characterized in that it additionally contains at least one cationic polymer, a softening agent, a protein hydrolysate, a wax, an opacifying agent, a fragrance, a dye, a non-ionic surface-active agent, an alcohol, a treatment agent or, alternatively, a penetrating agent.

15. Composition according to any one of Claims 7 to 14, characterized in that it additionally contains at least one disulphide, the composition being of the self-neutralizing type.

16. Composition according to Claim 15, characterized in that the disulphide is dithioglycolic acid, dithioglycerol, cystamine, N,N'-diacetylcystamine, cystine, pantethine or a disulphide of N-mercaptoalkyl-ω-hydroxyalkylamide or of N-mono- or N,N-dialkyl-4-mercaptobutyramide, a disulphide of aminomercaptoalkylamide, or a disulphide of derivatives of N-(mercaptoalkyl)succinamic acids or of N-(mercaptoalkyl)succinimides.

17. Composition according to Claim 15, characterized in that the disulphide corresponds to the following general formula (III) 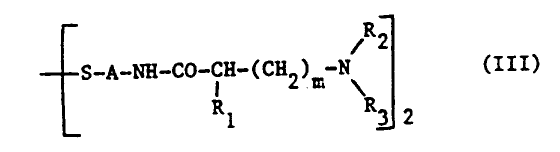 in which:
A, R₁, R₂, R₃ and m have the same meanings as those given in Claim 1 for the general formula (I), with the exception of the compound of formula (III) in which:
A = - (CH₂)₂-, m = 0,
R₁ and R₂ = H and R₃ = -C₂H₅.

18. Composition according to Claim 17, characterized in that the disulphide of formula (III) is chosen from:
N,N'-(dithiodi(2,1-ethanediyl))bis[2-dimethylaminoacetamide],
N,N'-(dithiodi(2,1-ethanediyl))bis[2-dimethylaminopropionamide],
N,N'-(dithiodi(2,1-ethanediyl))bis[3-dimethylaminopropionamide],
N,N'-(dithiodi(2,1-ethanediyl))bis[4-dimethylaminobutyramide],
N,N'-(dithiodi(2,1-ethanediyl))bis[2-methylaminoacetamide],
N,N'-[dithiobis(trimethylene)]bis[2-dimethylaminoacetamide],
N,N'-[dithiobis(pentamethylene)]bis[2-dimethylaminoacetamide],
N,N'-[dithiobis(2-ethoxyethyl)]bis[2-dimethylaminoacetamide],
N,N'-(dithiodi(2,1-ethanediyl))bis[3-methylaminopropionamide],
N,N'-(dithiodi(2,1-ethanediyl))bis[2-diethylaminoacetamide],
and N,N'-(dithiodi(2,1-ethanediyl))bis[2-ethylaminoacetamide].

19. Composition according to any one of Claims 15 to 18, characterized in that the disulphide is present in a molar proportion with respect to the compound of formula (I) ranging from 0.5 to 2.5 and preferably from 1 to 2.

20. Process for the permanent deformation of hair consisting, in a first step, in reducing the disulphide bonds of keratin by application of a reducing composition and then, in a second step, in reforming the said bonds by application of an oxidizing composition, characterized in that the reduction step is carried out using a cosmetic reducing composition as claimed according to any one of Claims 7 to 14.

21. Process according to Claim 20, characterized in that the reducing composition is left to act for a period of between 5 and 60 minutes.

## Claims (Claims for the following Contracting State(s): ES, GR)

1. Process for the preparation of alkylamino mercaptoalkylamides of following formula (I): 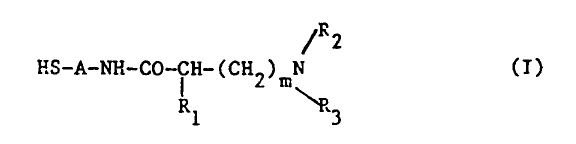 in which:
A represents the divalent radical -(CH₂)ₙ-,
n being an integer between 2 and 5, or the divalent radical -(CH₂)₂-O-(CH₂)₂-
m is equal to 0, 1 or 2
R₁ represents a hydrogen atom or a linear or branched lower alkyl radical having from 1 to 5 carbon atoms,
R₂ and R₃, which are identical or different, represent a hydrogen atom or a linear or branched lower alkyl radical having from 1 to 4 carbon atoms, it not being possible for R₂ and R₃ simultaneously to represent a hydrogen atom, the salts of the said compounds of formula (I) and their corresponding disulphides, with the exception of the disulphide in which:
A = -(CH₂)₂-, m = 0
R₁ and R₂ = H and R₃ = -C₂H₅, characterized in that it consists in reacting, in an inert solvent, an aminothiol of formula H₂N-A-SH with an acid halide of an alkylamino acid of formula: 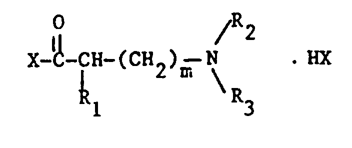 A, R₁, R₂, R₃ and m having the same meanings as those given in Claim 1, and X is Cl or Br, in order to obtain a thioester of formula: 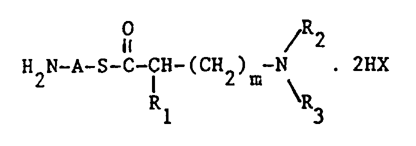 and in carrying out its rearrangement in aqueous/alcoholic medium in the presence of a base.

2. Cosmetic composition for the first step in an operation for the permanent deformation of hair, characterized in that it contains, in a suitable cosmetic vehicle, as reducing agent, at least one alkylamino mercaptoalkylamide of following formula (I): 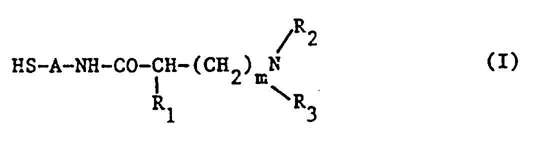 in which:
A represents the divalent radical -(CH₂)ₙ-,
n being an integer between 2 and 5, or the divalent radical -(CH₂)₂-O-(CH₂)₂-
m is equal to 0, 1 or 2
R₁ represents a hydrogen atom or a linear or branched lower alkyl radical having from 1 to 5 carbon atoms,
R₂ and R₃, which are identical or different, represent a hydrogen atom or a linear or branched lower alkyl radical having from 1 to 4 carbon atoms, it not being possible for R₂ and R₃ simultaneously to represent a hydrogen atom,
and the salts of the said compounds of formula (I).

3. Composition according to Claim 2, characterized in that the salts of the compounds of formula (I) are chosen from the hydrochlorides, hydrobromides, citrates, oxalates and acetates.

4. Composition according to Claim 2 or 3, characterized in that the compounds are chosen from:
- 2-dimethylamino-N-(2-mercaptoethyl)acetamide,
- 2-dimethylamino-N-(3-mercaptopropyl)acetamide,
- 2-dimethylamino-N-(5-mercaptopentyl)acetamide,
- 2-dimethylamino-N-[2'-(2-mercaptoethoxy)ethyl]acetamide,
- 2-diethylamino-N-(2-mercaptoethyl)acetamide,
- 2-diethylamino-N-(3-mercaptopropyl)acetamide,
- 2-diethylamino-N-(5-mercaptopentyl)acetamide,
- 2-diethylamino-N-[2'-(2-mercaptoethoxy)ethyl]acetamide,
- 2-methylamino-N-(2-mercaptoethyl)acetamide,
- 2-methylamino-N-(3-mercaptopropyl)acetamide,
- 2-methylamino-N-(5-mercaptopentyl)acetamide,
- 2-methylamino-N-[2'-(2-mercaptoethoxy)ethyl]acetamide,
- 2-ethylamino-N-(2-mercaptoethyl)acetamide,
- 2-ethylamino-N-(3-mercaptopropyl)acetamide,
- 2-ethylamino-N-(5-mercaptopentyl)acetamide,
- 2-ethylamino-N-[2'-(2-mercaptoethoxy)ethyl]acetamide,
- 2-ethylamino-2-methylamino-N-(2-mercaptoethyl)acetamide,
- 2-propylamino-N-(2-mercaptoethyl)acetamide,
- 2-isopropylamino-N-(2-mercaptoethyl)acetamide,
- 2-butylamino-N-(2-mercaptoethyl)acetamide,
- 2-ethylmethylamino-N-(2-mercaptoethyl)acetamide,
- 3-dimethylamino-N-(2-mercaptoethyl)propionamide,
- 3-dimethylamino-N-(3-mercaptopropyl)propionamide,
- 3-dimethylamino-N-(5-mercaptopentyl)propionamide,
- 3-dimethylamino-N-[2'-(2-mercaptoethoxy)ethyl]propionamide
- 3-methylamino-N-(2-mercaptoethyl)propionamide,
- 3-methylamino-N-(3-mercaptopropyl)propionamide,
- 3-methylamino-N-(5-mercaptopentyl)propionamide,
- 3-methylamino-N-[2'-(2-mercaptoethoxy)ethyl]propionamide,
- 2-dimethylamino-N-(2-mercaptoethyl)propionamide,
- 2-dimethylamino-N-(3-mercaptopropyl)propionamide,
- 2-dimethylamino-N-(5-mercaptopentyl)propionamide,
- 2-dimethylamino-N-[2'-(2-mercaptoethoxy)ethyl]propionamide,
- 2-methylamino-N-(2-mercaptoethyl)propionamide,
- 2-ethylamino-N-(2-mercaptoethyl)propionamide,
- 2-isopropylamino-N-(2-mercaptoethyl)propionamide,
- 2-methylamino-N-(2-mercaptoethyl)butanamide,
- 4-dimethylamino-N-(2-mercaptoethyl)butanamide,
- 4-dimethylamino-N-(3-mercaptopropyl)butanamide,
- 4-dimethylamino-N-(5-mercaptopentyl)butanamide,
- 4-dimethylamino-N-[2'-(2-mercaptoethoxy)ethyl]butanamide,
- 4-methylamino-N-(2-mercaptoethyl)butanamide,
- 4-ethylamino-N-(2-mercaptoethyl)butanamide,
- 2-dimethylamino-3-methyl-N-(2-mercaptoethyl)butanamide,
- 2-dimethylamino-4-methyl-N-(2-mercaptoethyl)pentanamide,
- 2-methylamino-4-methyl-N-(2-mercaptoethyl)pentanamide,
- 2-dimethylamino-3-methyl-N-(2-mercaptoethyl)pentanamide, and
- 2-methylamino-3-methyl-N-(2-mercaptoethyl)pentanamide.

5. Composition according to Claims 2 to 4, characterized in that the compound of formula (II) is formed in situ from its thioester precursor having the following general formula (II): 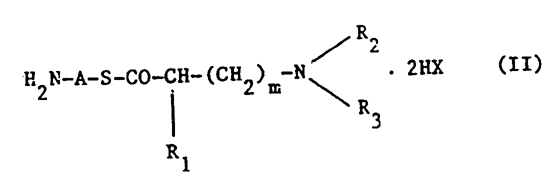 in which:
A, R₁, R₂, R₃ and m have the same meanings as those given in Claim 1 for the general formula (I), and
X is Cl or Br.

6. Composition according to any one of Claims 2 to 5, characterized in that the compound of formula (I) or (II) is present at a concentration of between 2 and 30% and preferably between 5 and 25% by weight with respect to the total weight of the composition.

7. Composition according to any one of Claims 2 to 6, characterized in that it has a pH of between 4.5 and 11, and preferably between 6 and 10, obtained using an alkaline agent chosen from ammonia, monoethanolamine, diethanolamine, triethanolamine, an alkali metal or ammonium carbonate or bicarbonate, or an alkali metal hydroxide or using an acidifying agent chosen from hydrochloric acid, acetic acid, lactic acid, oxalic acid or boric acid.

8. Composition according to any one of Claims 2 to 7, characterized in that it additionally contains at least one other reducing agent chosen from: thioglycolic acid, glycerol or glycol monothioglycolate, cysteamine and its C₁-C₄ acylated derivatives, cysteine, N-acetylcysteine, N-mercaptoalkylamides of sugar, β-mercaptopropionic acid and its derivatives, thiolactic acid, thiomalic acid, pantetheine, thioglycerol, an alkali metal or alkaline-earth metal sulphite or bisulphite, N-mercaptoalkyl-ω-hydroxyalkylamides, N-mono- or N,N-dialkyl-4-mercaptobutyramides, aminomercaptoalkylamides and derivatives of N-(mercaptoalkyl)succinamic acids or of N-(mercaptoalkyl)succinimides.

9. Composition according to any one of Claims 2 to 8, characterized in that it additionally contains at least one cationic polymer, a softening agent, a protein hydrolysate, a wax, an opacifying agent, a fragrance, a dye, a non-ionic surface-active agent, an alcohol, a treatment agent or, alternatively, a penetrating agent.

10. Composition according to any one of Claims 2 to 9, characterized in that it additionally contains at least one disulphide, the composition being of the self-neutralizing type.

11. Composition according to Claim 10, characterized in that the disulphide is dithioglycolic acid, dithioglycerol, cystamine, N,N'-diacetylcystamine, cystine, pantethine or a disulphide of N-mercaptoalkyl-ω-hydroxyalkylamide or of N-mono- or N,N-dialkyl-4-mercaptobutyramide, a disulphide of aminomercaptoalkylamide, or a disulphide of derivatives of N-(mercaptoalkyl)succinamic acids or of N-(mercaptoalkyl)succinimides.

12. Composition according to Claim 11, characterized in that the disulphide corresponds to the following general formula (III) 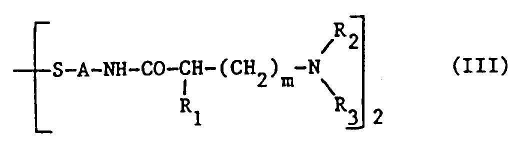 in which:
A, R₁, R₂, R₃ and m have the same meanings as those given in Claim 1 for the general formula (I), with the exception of the compound of formula (III) in which:
A = -(CH₂)₂-, m = 0,
R₁ and R₂ = H and R₃ = -C₂H₅.

13. Composition according to Claim 12, characterized in that the disulphide of formula (III) is chosen from:
N,N'-(dithiodi(2,1-ethanediyl))bis[2-dimethylaminoacetamide],
N,N'-(dithiodi(2,1-ethanediyl))bis[2-dimethylaminopropionamide],
N,N'-(dithiodi(2,1-ethanediyl))bis[3-dimethylaminopropionamide],
N,N'-(dithiodi(2,1-ethanediyl))bis[4-dimethylaminobutyramide],
N,N'-(dithiodi(2,1-ethanediyl))bis[2-methylaminoacetamide],
N,N'-[dithiobis(trimethylene)]bis[2-dimethylaminoacetamide],
N,N'-[dithiobis(pentamethylene)]bis[2-dimethylaminoacetamide],
N,N'-[dithiobis(2-ethoxyethyl)]bis[2-dimethylaminoacetamide],
N,N'-(dithiodi(2,1-ethanediyl))bis[3-methylaminopropionamide],
N,N'-(dithiodi(2,1-ethanediyl))bis[2-diethylaminoacetamide],
and N,N'-(dithiodi(2,1-ethanediyl))bis[2-ethylaminoacetamide].

14. Composition according to any one of Claims 10 to 13, characterized in that the disulphide is present in a molar proportion with respect to the compound of formula (I) ranging from 0.5 to 2.5 and preferably from 1 to 2.

15. Process for the permanent deformation of hair consisting, in a first step, in reducing the disulphide bonds of keratin by application of a reducing composition and then, in a second step, in reforming the said bonds by application of an oxidizing composition, characterized in that the reduction step is carried out using a cosmetic reducing composition as claimed according to any one of Claims 2 to 9.

16. Process according to Claim 15, characterized in that the reducing composition is left to act for a period of between 5 and 60 minutes.

## Patentansprüche (Patentansprüche für folgende(n) Vertragsstaat(en): AT, BE, CH, DE, DK, FR, GB, IT, LI, NL, PT, SE)

1. Neue Verbindungen, dadurch **gekennzeichnet**, daß sie der folgenden allgemeinen Formel (I) entsprechen: 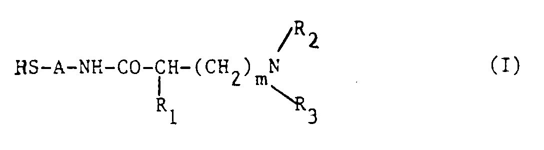 worin:
A den zweiwertigen Rest -(CH₂)ₙ-
n eine ganze Zahl von 2 bis 5 oder den zweiwertigen Rest -(CH₂)₂-O-(CH₂)₂- bedeuten
m gleich 0, 1 oder 2 bedeutet,
R₁ ein Wasserstoffatom oder einen geradkettigen oder verzweigtkettigen niederen Alkylrest mit 1 bis 5 Kohlenstoffatomen darstellt,
R₂ und R₃, die gleich oder verschieden sein können, ein Wasserstoffatom oder einen gerad- oder verzweigtkettigen niedrigen Alkylrest mit 1 bis 4 Kohlenstoffatomen bedeuten, wobei R₂ und R₃ nicht gleichzeitig ein Wasserstoffatom darstellen,
die Salze der Verbindungen gemäß Formel (I) wie auch deren Disulfide, jedoch mit der Maßgabe, daß Disulfide ausgeschlossen sind, worin:
A = -(CH₂)₂-, m = 0,
R₁ und R₂ = H und R₃ = -C₂H₅ bedeuten.

2. Verbindungen gemäß Anspruch 1, dadurch **gekennzeichnet**, daß sie unter den folgenden Verbindungen ausgewählt sind:
Dimethylamino-2-N-(2-mercaptoethyl)-acetamid,
Dimethylamino-2-N-(3-mercaptopropyl)-acetamid,
Dimethylamino-2-N-(5-mercaptopentyl)-acetamid,
Dimethylamino-2-N-[2'-ethyl-(2-mercaptoethoxy)]-acetamid,
Diethylamino-2-N-(2-mercaptoethyl)-acetamid,
Diethylamino-2-N-(3-mercaptopropyl)-acetamid,
Diethylamino-2-N-(5-mercaptopentyl)-acetamid,
Diethylamino-2-N-[2'-ethyl-(2-mercaptoethoxy)]-acetamid,
Methylamino-2-N-(2-mercaptoethyl)-acetamid,
Methylamino-2-N-(3-mercaptopropyl)-acetamid,
Methylamino-2-N-(5-mercaptopentyl)-acetamid,
Methylamino-2-N-[2'-ethyl-(2-mercaptoethoxy)]-acetamid,
Ethylamino-2-N-(2-mercaptoethyl)-acetamid,
Ethylamino-2-N-(3-mercaptopropyl)-acetamid,
Ethylamino-2-N-(5-mercaptopentyl)-acetamid,
Ethylamino-2-N-[2'-ethyl-(2-mercaptoethoxy)]-acetamid,
Ethylamino-2-methylamino-2-N-(2-mercaptoethyl)-acetamid,
Propylamino-2-N-(2-mercaptoethyl)-acetamid,
Isopropylamino-2-N-(2-mercaptoethyl)-acetamid,
Butylamino-2-N-(2-mercaptoethyl)-acetamid,
Ethylmethylamino-2-N-(2-mercaptoethyl)-acetamid,
Dimethylamino-3-N-(2-mercaptoethyl)-propionamid,
Dimethylamino-3-N-(3-mercaptopropyl)-propionamid,
Dimethylamino-3-N-(5-mercaptopentyl)-propionamid,
Dimethylamino-3-N-[2'-ethyl-(2-mercaptoethoxy)]-propionamid,
Methylamino-3-N-(2-mercaptoethyl)-propionamid,
Methylamino-3-N-(3-mercaptopropyl)-propionamid,
Methylamino-3-N-(5-mercaptopentyl)-propionamid,
Methylamino-3-N-[2'-ethyl-(2-mercaptoethoxy)]-propionamid,
Dimethylamino-2-N-(2-mercaptoethyl)-propionamid,
Dimethylamino-2-N-(3-mercaptopropyl)-propionamid,
Dimethylamino-2-N-(5-mercaptopentyl)-propionamid,
Dimethylamino-2-N-[2'-ethyl-(2-mercaptoethoxy)]-propionamid,
Methylamino-2-N-(2-mercaptoethyl)-propionamid,
Ethylamino-2-N-(2-mercaptoethyl)-propionamid,
Isopropyl-2-N-(2-mercaptoethyl)-propionamid,
Methylamino-2-N-(2-mercaptoethyl)-butanamid,
Dimethylamino-4-N-(2-mercaptoethyl)-butanamid,
Dimethylamino-4-N-(3-mercaptopropyl)-butanamid,
Dimethylamino-4-N-(5-mercaptopentyl)-butanamid,
Dimethylamino-4-N-[2'-ethyl-(2-mercaptoethoxy)]-butanamid,
Methylamino-4-N-(2-mercaptoethyl)-butanamid,
Ethylamino-4-N-(2-mercaptoethyl)-butanamid,
Dimethylamino-2-methyl-3-N-(2-mercaptoethyl)-butanamid,
Dimethylamino-2-methyl-4-N-(2-mercaptoethyl)-pentanamid,
Methylamino-2-methyl-4-N-(2-mercaptoethyl)-pentanamid,
Dimethylamino-2-methyl-3-N-(2-mercaptoethyl)-pentanamid sowie
Methylamino-2-methyl-3-N-(2-mercaptoethyl)-pentanamid.

3. Verbindungen nach Anspruch 1, dadurch **gekennzeichnet**, daß die Disulfide unter den folgenden Verbindungen ausgewählt sind:
N,N'-(2,1-Dithiodiethandiyl)-bis-[2-dimethylaminoacetamid],
N,N'-(2,1-Dithiodiethandiyl)-bis-[2-dimethylaminopropionamid],
N,N'-(2,1-Dithiodiethandiyl)-bis-[3-dimethylaminopropionamid],
N,N'-(2,1-Dithiodiethandiyl)-bis-[4-dimethylaminobutyramid],
N,N'-(2,1-Dithiodiethandiyl)-bis-[2-methylaminoacetamid]
N,N'-[Dithiobis-(trimethylen)]-bis-[2-dimethylaminoacetamid]
N,N'-[Dithiobis-(pentamethylen)]-bis-[2-dimethylaminoacetamid]
N,N'-[Dithiobis-(2-ethoxyethyl)]-bis-[2-dimethylaminoacetamid]
N,N'-(2,1-Dithiodiethandiyl)-bis-[3-methylaminopropionamid],
N,N'-(2,1-Dithiodiethandiyl)-bis-[2-diethylaminoacetamid] sowie
N,N'-(2,1-Dithiodiethandiyl)-bis-[2-ethylaminoacetamid].

4. Neue Verbindungen, dadurch **gekennzeichnet**, daß sie der folgenden allgemeinen Formel (II) entsprechen: 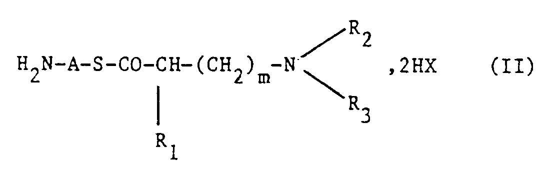 worin:
A, R₁, R₂ und R₃ sowie m der vorstehend genannten Bedeutung gemäß Anspruch 1 im Hinblick auf die allgemeine Formel (I) entsprechen,
wobei X Cl oder Br darstellt.

5. Verbindungen nach Anspruch 4, dadurch **gekennzeichnet**, daß sie unter den folgenden Verbindungen ausgewählt sind:
2-Aminoethyl-N,N-dimethylthioglycinat-dichlorhydrat,
3-Aminopropyl-N,N-dimethylthioglycinat-dichlorhydrat,
5-Aminopentyl-N,N-dimethylthioglycinat-dichlorhydrat,
2'-Ethyl-(2-aminoethoxy)-N,N-dimethylthioglycinat-dichlorhydrat,
2-Aminoethyl-N,N-diethylthioglycinat-dichlorhydrat,
3-Aminopropyl-N,N-diethylthioglycinat-dichlorhydrat,
5-Aminopentyl-N,N-diethylthioglycinat-dichlorhydrat,
2'-Ethyl-(2-aminoethoxy)-N,N-diethylthioglycinat-dichlorhydrat,
2-Aminoethyl-N-methylthioglycinat-dichlorhydrat,
3-Aminopropyl-N-methylthioglycinat-dichlorhydrat,
5-Aminopentyl-N-methylthioglycinat-dichlorhydrat,
2'-Ethyl-(2-aminoethoxy)-N-methylthioglycinat-dichlorhydrat,
2-Aminoethyl-N-ethylthioglycinat-dichlorhydrat,
3-Aminopropyl-N-ethylthioglycinat-dichlorhydrat,
5-Aminopentyl-N-ethylthioglycinat-dichlorhydrat,
2'-Ethyl-(2-aminoethoxy)-N-ethylthioglycinat-dichlorhydrat,
2-Aminoethyl-N-ethyl-N-methyl-thioglycinat-dichlorhydrat,
2-Aminoethyl-N-propylthioglycinat-dichlorhydrat,
2-Aminoethyl-N-isopropylthioglycinat-dichlorhydrat,
2-Aminoethyl-N-butylthioglycinat-dichlorhydrat,
2-Aminoethyl-3-dimethylaminothiopropionat-dichlorhydrat,
3-Aminopropyl-3-dimethylaminothiopropionat-dichlorhydrat,
5-Aminopentyl-3-dimethylaminothiopropionat-dichlorhydrat,
2'-Ethyl-(2-aminoethoxy)-3-dimethylaminothiopropionat-dichlorhydrat,
2-Aminoethyl-3-methylaminothiopropionat-dichlorhydrat,
3-Aminopropyl-3-methylaminothiopropionat-dichlorhydrat,
5-Aminopentyl-3-methylaminothiopropionat-dichlorhydrat,
2'-Ethyl-(2-aminoethoxy)-3-methylaminothiopropionat-dichlorhydrat,
2-Aminoethyl-N,N-dimethylthioalaninat-dichlorhydrat,
3-Aminopropyl-N,N-dimethylthioalaninat-dichlorhydrat,
5-Aminopentyl-N,N-dimethylthioalaninat-dichlorhydrat,
2'-Ethyl-(2-aminoethoxy)-N,N-dimethylthioalaninat-dichlorhydrat,
2-Aminoethyl-N-methylthioalaninat-dichlorhydrat,
2-Aminoethyl-N-ethylthioalaninat-dichlorhydrat,
2-Aminoethyl-N-isopropylthioalaninat-dichlorhydrat,
2-Aminoethyl-N-methylthiobutyrat-dichlorhydrat,
2-Aminoethyl-4-dimethylaminothiobutyrat-dichlorhydrat,
3-Aminopropyl-4-dimethylaminothiobutyrat-dichlorhydrat,
5-Aminopentyl-4-dimethylaminothiobutyrat-dichlorhydrat,
2'-Ethyl-(2-aminoethoxy)-4-dimethylaminothiobutyrat-dichlorhydrat,
2-Aminoethyl-4-methylaminothiobutyrat-dichlorhydrat,
2-Aminoethyl-4-ethylaminothiobutyrat-dichlorhydrat,
2-Aminoethyl-N,N-dimethylthiovalinat-dichlorhydrat,
2-Aminoethyl-N,N-dimethylaminothioleucinat-dichlorhydrat,
2-Aminoethyl-N-methylaminothioleucinat-dichlorhydrat,
2-Aminoethyl-N,N-dimethylaminothioisoleucinat-dichlorhydrat,
2-Aminoethyl-N-methylaminothioleuzinat-dichlorhydrat,
2-Aminoethyl-thiopipecolinat-dichlorhydrat sowie das
2-Aminoethyl-hexahydroisothionicotinat-dichlorhydrat.

6. Verfahren zur Herstellung von Alkylaminomercaptoalkylamiden gemäß Formel (I) nach Anspruch 1 oder 2, dadurch **gekennzeichnet**, daß es darin besteht, daß man in einem inerten Lösungsmittel ein Aminothiol gemäß der Formel H₂N-A-SH mit einem Alkylaminosäure-Säurehalogenid gemäß der folgenden Formel reagieren läßt: 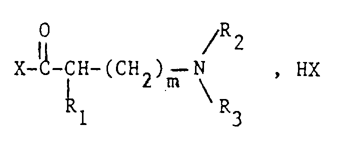 wobei A, R₁, R₂, R₃ und m jeweils dieselbe Bedeutung aufweisen, wie sie in Anspruch 1 definiert ist, wobei X Cl oder Br darstellt, zur Synthese eines Thioesters der folgenden Formel: 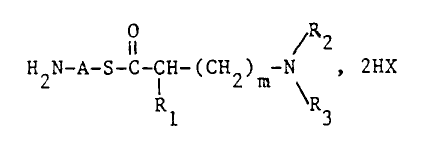 wobei dessen Umgruppierung in einem wäßrig-alkoholischen Medium in Anwesenheit einer Base bewerkstelligt wird.

7. Kosmetische Zubereitung für den ersten Arbeitsgang einer beständigen Verformung der Haare zur Dauerwelle, dadurch **gekennzeichnet**, daß sie in einem geeigneten kosmetischen Träger als Reduktionsmittel mindestens ein Alkylaminomercaptoalkylamid der folgenden allgemeinen Formel enthält: 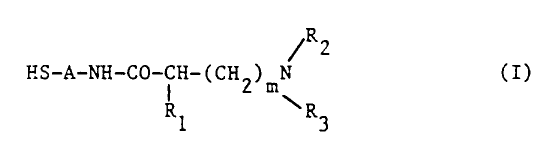 worin
A einen zweiwertigen Rest -(CH₂)ₙ-,
n eine ganze Zahl von 2 bis 5 oder die zweiwertige Gruppe -(CH₂)₂-O-(CH₂)₂- bedeuten, und
m gleich 0, 1 oder 2
R₁ ein Wasserstoffatom oder einen gerad- oder verzweigtkettigen niederen Alkylrest mit 1 bis 5 Kohlenstoffatomen darstellt,
R₂ und R₃, die gleich oder verschieden sein können, ein Wasserstoffatom oder einen gerad- oder verzweigtkettigen niedrigen Alkylrest mit 1 bis 4 Kohlenstoffatomen bedeuten, wobei R₂ und R₃ nicht gleichzeitig ein Wasserstoffatom darstellen können,
sowie die Salze von Verbindungen gemäß Formel (I).

8. Zubereitung nach Anspruch 7, dadurch **gekennzeichnet**, daß die Salze der Verbindungen gemäß Formel (I) unter Chlorhydraten, Bromhydraten, Citraten, Oxalaten und Acetaten ausgewählt sind.

9. Zubereitung nach Anspruch 7 oder 8, dadurch **gekennzeichnet**, daß die Verbindungen unter den folgenden Stoffen ausgewählt sind:
Dimethylamino-2-N-(2-mercaptoethyl)-acetamid,
Dimethylamino-2-N-(3-mercaptopropyl)-acetamid,
Dimethylamino-2-N-(5-mercaptopentyl)-acetamid,
Dimethylamino-2-N-[2'-ethyl-(2-mercaptoethoxy)]-acetamid,
Diethylamino-2-N-(2-mercaptoethyl)-acetamid,
Diethylamino-2-N-(3-mercaptopropyl)-acetamid,
Diethylamino-2-N-(5-mercaptopentyl)-acetamid,
Diethylamino-2-N-[2'-ethyl-(2-mercaptoethoxy)]-acetamid,
Methylamino-2-N-(2-mercaptoethyl)-acetamid,
Methylamino-2-N-(3-mercaptopropyl)-acetamid,
Methylamino-2-N-(5-mercaptopentyl)-acetamid,
Methylamino-2-N-[2'-ethyl-(2-mercaptoethoxy)]-acetamid,
Ethylamino-2-N-(2-mercaptoethyl)-acetamid,
Ethylamino-2-N-(3-mercaptopropyl)-acetamid,
Ethylamino-2-N-(5-mercaptopentyl)-acetamid,
Ethylamino-2-N-[2'-ethyl-(2-mercaptoethoxy)]-acetamid,
Ethylamino-2-methylamino-2-N-(2-mercaptoethyl)-acetamid,
Propylamino-2-N-(2-mercaptoethyl)-acetamid,
Isopropylamino-2-N-(2-mercaptoethyl)-acetamid,
Butylamino-2-N-(2-mercaptoethyl)-acetamid,
Ethylmethylamino-2-N-(2-mercaptoethyl)-acetamid,
Dimethylamino-3-N-(2-mercaptoethyl)-propionamid,
Dimethylamino-3-N-(3-mercaptopropyl)-propionamid,
Dimethylamino-3-N-(5-mercaptopentyl)-propionamid,
Dimethylamino-3-N-[2'-ethyl-(2-mercaptoethoxy)]-propionamid,
Methylamino-3-N-(2-mercaptoethyl)-propionamid,
Methylamino-3-N-(3-mercaptopropyl)-propionamid,
Methylamino-3-N-(5-mercaptopentyl)-propionamid,
Methylamino-3-N-[2'-ethyl-(2-mercaptoethoxy)]-propionamid,
Dimethylamino-2-N-(2-mercaptoethyl)-propionamid,
Dimethylamino-2-N-(3-mercaptopropyl)-propionamid,
Dimethylamino-2-N-(5-mercaptopentyl)-propionamid,
Dimethylamino-2-N-[2'-ethyl-(2-mercaptoethoxy)]-propionamid,
Methylamino-2-N-(2-mercaptoethyl)-propionamid,
Ethylamino-2-N-(2-mercaptoethyl)-propionamid,
Isopropyl-2-N-(2-mercaptoethyl)-propionamid,
Methylamino-2-N-(2-mercaptoethyl)-butanamid,
Dimethylamino-4-N-(2-mercaptoethyl)-butanamid,
Dimethylamino-4-N-(3-mercaptopropyl)-butanamid,
Dimethylamino-4-N-(5-mercaptopentyl)-butanamid,
Dimethylamino-4-N-[2'-ethyl-(2-mercaptoethoxy)]-butanamid,
Methylamino-4-N-(2-mercaptoethyl)-butanamid,
Ethylamino-4-N-(2-mercaptoethyl)-butanamid,
Dimethylamino-2-methyl-3-N-(2-mercaptoethyl)-butanamid,
Dimethylamino-2-methyl-4-N-(2-mercaptoethyl)-pentanamid,
Methylamino-2-methyl-4-N-(2-mercaptoethyl)-pentanamid,
Dimethylamino-2-methyl-3-N-(2-mercaptoethyl)-pentanamid sowie
Methylamino-2-methyl-3-N-(2-mercaptoethyl)-pentanamid.

10. Zubereitung nach den Ansprüchen 7 bis 9, dadurch **gekennzeichnet**, daß die Verbindung gemäß Formel (I) in situ ausgehend von der Thioester-Vorläuferverbindung mit der folgenden allgemeinen Formel (II) synthetisiert wird: 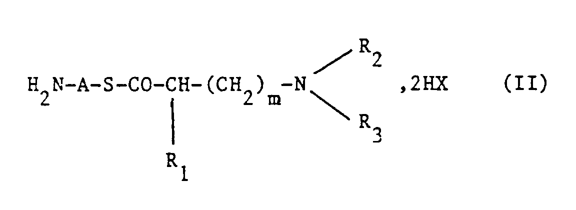 worin
A, R₁, R₂, R₃ und m die in Anspruch 1 angegebene Bedeutung wie für die allgemeine Formel (I) aufweisen,
wobei X Cl oder Br darstellt.

11. Zubereitung nach einem der Ansprüche 7 bis 10, dadurch **gekennzeichnet**, daß die Verbindung gemäß den Formeln (I) oder (II) in einer Konzentration zwischen 2 und 30 Gew.-%, vorzugsweise zwischen 5 und 25 Gew.-%, in bezug auf das Gesamtgewicht der Zusammensetzung enthalten ist.

12. Zubereitung nach einem der Ansprüche 7 bis 11, dadurch **gekennzeichnet**, daß sie einen pH-Wert zwischen 4,5 und 11, vorzugsweise zwischen 6 und 10, aufweist, der mit Hilfe eines alkalischen Mittels erzielt worden ist, das unter Ammoniak, Monoethanolamin, Diethanolamin, Triethanolamin, Alkali- oder Ammoniumkarbonat bzw. -hydrogenkarbonat, einem Alkalihydroxid ausgewählt wurde, oder wobei der pH-Wert mittels eines Säuerungsmittels eingestellt worden ist, das unter Salzsäure, Essigsäure, Milchsäure, Oxal- oder Borsäure ausgewählt wurde.

13. Zubereitung nach einem der Ansprüche 7 bis 12, dadurch **gekennzeichnet**, daß sie zusätzlich noch mindestens ein weiteres Reduktionsmittel enthält, das unter den folgenden Stoffen ausgewählt wurde: Thioglykolsäure, Glycerin- oder Glykolmonothioglykolat, Cysteamin und dessen acylierte C₁-C₄-Derivate, Cystein, N-Acetylcystein, Zucker-N-Mercaptoalkylamide, β-Mercaptiopropionsäure und deren Derivate, Thiomilchsäure, Thioäpfelsäure, Pantethein, Thioglycerin, Alkali- oder Erdalkalisulfit oder -bisulfit, N-(Mercaptoalkyl)-ω-hydroxyalkylamide, N-Mono- oder N,N-4-Dialkylmercaptobutyramide, Aminomercaptoalkylamide und Derivate von N-(Mercaptoalkyl)-bernsteinsäureamiden oder Derivate von N-(Mercaptoalkyl)-succinimiden.

14. Zubereitung nach einem der Ansprüche 7 bis 13, dadurch **gekennzeichnet**, daß sie zusätzlich noch mindestens ein kationisches Polymer, ein Erweichungsmittel, Proteinhydrolysat, Wachs, einen Opakmacher, ein Parfüm, einen Farbstoff, ein nichtionisches Tensid, einen Alkohol, ein Behandlungsmittel (Wirkstoff) oder auch noch ein Penetriermittel enthält.

15. Zubereitung nach einem der Ansprüche 7 bis 14, dadurch **gekennzeichnet**, daß sie zusätzlich noch mindestens ein Disulfid enthält, wobei die Zubereitung einen selbstneutralisierenden Typus darstellt.

16. Zubereitung nach Anspruch 15, dadurch **gekennzeichnet**, daß das Disulfit eine Dithioglykolsäure, ein Dithioglycerin, Cystamin, N,N'-Diacetylcystamin, das Cystin, Pantethin oder ein N-(Mercaptoalkyl)-ω-hydroxyalkylamiddisulfid oder ein N-Mono- oder N,N-Dialkyl-4-mercaptobutyramid-disulfit, ein Aminomercaptoalkylamiddisulfid oder ein Disulfid von Derivaten von N-(Mercaptoalkyl)-Succinamiden oder N-(Mercaptoalkyl)-Succinimiden.

17. Zubereitung nach Anspruch 15, dadurch **gekennzeichnet**, daß das Disulfid der folgenden allgemeinen Formel (III) entspricht: 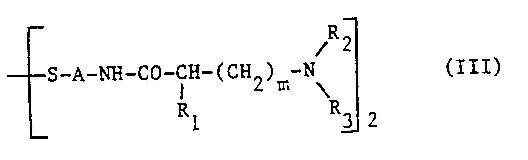 A, R₁, R₂ und R₃ sowie m die in Anspruch 1 im Hinblick auf die allgemeine Formel (I) angegebenen Bedeutungen aufweisen, wobei Verbindungen der Formel (III) ausgenommen sind, worin
A = -(CH₂)₂-, m = 0
R₁ und R₂ = H und R₃ = -C₂H₅ bedeuten.

18. Zubereitung nach Anspruch 17, dadurch **gekennzeichnet**, daß das Disulfid gemäß der Formel (III) unter den folgenden Stoffen ausgewählt ist:
N,N'-(2,1-Dithiodiethandiyl)-bis-[2-dimethylaminoacetamid],
N,N'-(2,1-Dithiodiethandiyl)-bis-[2-dimethylaminopropionamid],
N,N'-(2,1-Dithiodiethandiyl)-bis-[3-dimethylaminopropionamid],
N,N'-(2,1-Dithiodiethandiyl)-bis-[4-dimethylaminobutyramid],
N,N'-(2,1-Dithiodiethandiyl)-bis-[2-methylaminoacetamid]
N,N'-[Dithiobis-(trimethylen)]-bis-[2-dimethylaminoacetamid]
N,N'-[Dithiobis-(pentamethylen)]-bis-[2-dimethylaminoacetamid]
N,N'-[Dithiobis-(2-ethoxyethyl)]-bis-[2-dimethylaminoacetamid]
N,N'-(2,1-Dithiodiethandiyl)-bis-[3-methylaminopropionamid],
N,N'-(2,1-Dithiodiethandiyl)-bis-[2-diethylaminoacetamid] sowie
N,N'-(2,1-Dithiodiethandiyl)-bis-[2-ethylaminoacetamid].

19. Zubereitung nach einem der Ansprüche 15 bis 18, dadurch **gekennzeichnet**, daß das Disulfid in einem molaren Verhältnis in bezug auf die Verbindung gemäß der Formel (I) von 0,5 bis 2,5, vorzugsweise von 1 bis 2, vorhanden ist.

20. Verfahren zur dauerhaften Verformung der Haare (Dauerwelle), das darin besteht, daß in einem ersten Arbeitsschritt zur Reduktion der Disulfidbindungen des Keratins eine reduzierende Zusammensetzung appliziert und im Anschluß daran in einem zweiten Schritt zur Wiederherstellung dieser Bindungen eine oxidierende Zusammensetzung appliziert wird, dadurch **gekennzeichnet**, daß der Reduktionsschritt mit Hilfe einer reduzierenden kosmetischen Zubereitung bewerkstelligt wird, wie sie nach einem der Ansprüche 7 bis 14 beansprucht ist.

21. Verfahren nach Anspruch 20, dadurch **gekennzeichnet**, daß man die reduzierende Zubereitung während der Zeitdauer zwischen 5 und 60 Minuten einwirken läßt.

## Patentansprüche (Patentansprüche für folgende(n) Vertragsstaat(en): ES, GR)

1. Verfahren zur Herstellung von Alkylaminomercaptoalkylamiden gemäß der folgenden Formel (I): 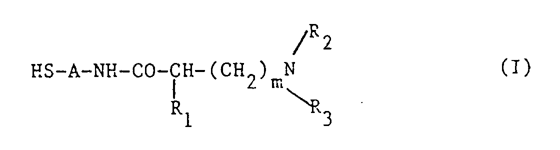 worin:
A den zweiwertigen Rest -(CH₂)ₙ-
n eine ganze Zahl von 2 bis 5 oder den zweiwertigen Rest -(CH₂)₂-O-(CH₂)₂- bedeuten
m gleich 0, 1 oder 2 bedeutet,
R₁ ein Wasserstoffatom oder einen geradkettigen oder verzweigtkettigen niederen Alkylrest mit 1 bis 5 Kohlenstoffatomen darstellt,
R₂ und R₃, die gleich oder verschieden sein können, ein Wasserstoffatom oder einen gerad- oder verzweigtkettigen niedrigen Alkylrest mit 1 bis 4 Kohlenstoffatomen bedeuten, wobei R₂ und R₃ nicht gleichzeitig ein Wasserstoffatom bedeuten können,
die Salze der Verbindungen gemäß Formel (I) wie auch deren Disulfide, worin:
A = -(CH₂)₂-, m = 0,
R₁ und R₂ = H und R₃ = -C₂H₅ bedeuten,
dadurch **gekennzeichnet**, daß man in einem inerten Lösungsmittel ein Aminothiol gemäß der Formel H₂N-A-SH mit einem Alkylaminosäurehalogenid gemäß der folgenden Formel reagieren läßt: 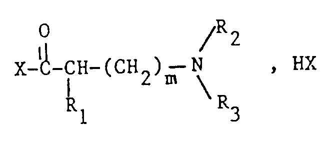 wobei A, R₁, R₂, R₃ und m die in Anspruch 1 angegebenen Bedeutungen aufweisen und wobei X Cl oder Br darstellt, zur Synthese eines Thioesters der folgenden Formel: 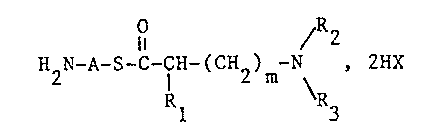 wobei deren Umgruppierung in einem wäßrig-alkoholischen Medium in Anwesenheit einer Base bewerkstelligt wird.

2. Kosmetische Zubereitung für den ersten Arbeitsschritt bei der dauerhaften Verformung der Haare zur Dauerwelle, dadurch **gekennzeichnet**, daß sie in einem geeigneten kosmetischen Träger als Reduktionsmittel mindestens ein Alkylaminomercaptoalkylamid der folgenden allgemeinen Formel enthält: 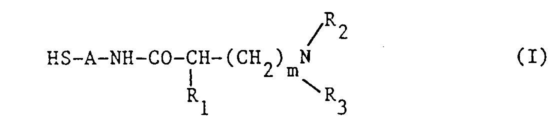 worin
A einen zweiwertigen Rest -(CH₂)ₙ-,
n eine ganze Zahl von 2 bis 5
oder die zweiwertige Gruppe -(CH₂)₂-O-(CH₂)₂- bedeuten, und
m ist gleich 0, 1 oder 2,
R₁ ein Wasserstoffatom oder einen gerad- oder verzweigtkettigen niederen Alkylrest mit 1 bis 5 Kohlenstoffatomen darstellt,
R₂ und R₃, die gleich oder verschieden sein können, ein Wasserstoffatom oder einen gerad- oder verzweigtkettigen niedrigen Alkylrest mit 1 bis 4 Kohlenstoffatomen bedeuten, wobei R₂ und R₃ nicht gleichzeitig ein Wasserstoffatom bedeuten können,
sowie die Salze von Verbindungen gemäß Formel (I).

3. Zubereitung nach Anspruch 2, dadurch **gekennzeichnet**, daß die Salze der Verbindungen gemäß Formel (I) unter Chlorhydraten, Bromhydraten, Citraten, Oxalaten und Acetaten ausgewählt sind.

4. Zubereitung nach Anspruch 2 oder 3, dadurch **gekennzeichnet**, daß die Verbindungen unter den folgenden Stoffen ausgewählt sind:
Dimethylamino-2-N-(2-mercaptoethyl)-acetamid,
Dimethylamino-2-N-(3-mercaptopropyl)-acetamid,
Dimethylamino-2-N-(5-mercaptopentyl)-acetamid,
Dimethylamino-2-N-[2'-ethyl-(2-mercaptoethoxy)]-acetamid,
Diethylamino-2-N-(2-mercaptoethyl)-acetamid,
Diethylamino-2-N-(3-mercaptopropyl)-acetamid,
Diethylamino-2-N-(5-mercaptopentyl)-acetamid,
Diethylamino-2-N-[2'-ethyl-(2-mercaptoethoxy)]-acetamid,
Methylamino-2-N-(2-mercaptoethyl)-acetamid,
Methylamino-2-N-(3-mercaptopropyl)-acetamid,
Methylamino-2-N-(5-mercaptopentyl)-acetamid,
Methylamino-2-N-[2'-ethyl-(2-mercaptoethoxy)]-acetamid,
Ethylamino-2-N-(2-mercaptoethyl)-acetamid,
Ethylamino-2-N-(3-mercaptopropyl)-acetamid,
Ethylamino-2-N-(5-mercaptopentyl)-acetamid,
Ethylamino-2-N-[2'-ethyl-(2-mercaptoethoxy)]-acetamid,
Ethylamino-2-methylamino-2-N-(2-mercaptoethyl)-acetamid,
Propylamino-2-N-(2-mercaptoethyl)-acetamid,
Isopropylamino-2-N-(2-mercaptoethyl)-acetamid,
Butylamino-2-N-(2-mercaptoethyl)-acetamid,
Ethylmethylamino-2-N-(2-mercaptoethyl)-acetamid,
Dimethylamino-3-N-(2-mercaptoethyl)-propionamid,
Dimethylamino-3-N-(3-mercaptopropyl)-propionamid,
Dimethylamino-3-N-(5-mercaptopentyl)-propionamid,
Dimethylamino-3-N-[2'-ethyl-(2-mercaptoethoxy)]-propionamid,
Methylamino-3-N-(2-mercaptoethyl)-propionamid,
Methylamino-3-N-(3-mercaptopropyl)-propionamid,
Methylamino-3-N-(5-mercaptopentyl)-propionamid,
Methylamino-3-N-[2'-ethyl-(2-mercaptoethoxy)]-propionamid,
Dimethylamino-2-N-(2-mercaptoethyl)-propionamid,
Dimethylamino-2-N-(3-mercaptopropyl)-propionamid,
Dimethylamino-2-N-(5-mercaptopentyl)-propionamid,
Dimethylamino-2-N-[2'-ethyl-(2-mercaptoethoxy)]-propionamid,
Methylamino-2-N-(2-mercaptoethyl)-propionamid,
Ethylamino-2-N-(2-mercaptoethyl)-propionamid,
Isopropyl-2-N-(2-mercaptoethyl)-propionamid,
Methylamino-2-N-(2-mercaptoethyl)-butanamid,
Dimethylamino-4-N-(2-mercaptoethyl)-butanamid,
Dimethylamino-4-N-(3-mercaptopropyl)-butanamid,
Dimethylamino-4-N-(5-mercaptopentyl)-butanamid,
Dimethylamino-4-N-[2'-ethyl-(2-mercaptoethoxy)]-butanamid,
Methylamino-4-N-(2-mercaptoethyl)-butanamid,
Ethylamino-4-N-(2-mercaptoethyl)-butanamid,
Dimethylamino-2-methyl-3-N-(2-mercaptoethyl)-butanamid,
Dimethylamino-2-methyl-4-N-(2-mercaptoethyl)-pentanamid,
Methylamino-2-methyl-4-N-(2-mercaptoethyl)-pentanamid,
Dimethylamino-2-methyl-3-N-(2-mercaptoethyl)-pentanamid sowie
Methylamino-2-methyl-3-N-(2-mercaptoethyl)-pentanamid.

5. Zubereitung nach den Ansprüchen 2 bis 4, dadurch **gekennzeichnet**, daß die Verbindung gemäß Formel (I) in situ ausgehend von dessen Thioester-Vorläufern der folgenden allgemeinen Formel (II) synthetisiert wird: 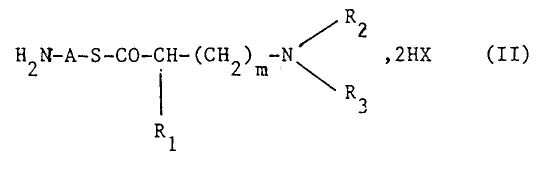 worin
A, R₁, R₂, R₃ und m die in Anspruch 1 für die allgemeine Formel (I) angegebene Bedeutung aufweisen,
wobei X Cl oder Br darstellt.

6. Zubereitung nach einem der Ansprüche 2 bis 5, dadurch **gekennzeichnet**, daß die Verbindung gemäß den Formeln (I) oder (II) in einer Konzentration zwischen 2 und 30 Gew.-%, vorzugsweise zwischen 5 und 25 Gew.-%, in bezug auf das Gesamtgewicht der Zusammensetzung enthalten ist.

7. Zubereitung nach einem der Ansprüche 2 bis 6, dadurch **gekennzeichnet**, daß sie einen pH-Wert zwischen 4,5 und 11, vorzugsweise zwischen 6 und 10, aufweist, wobei die pH-Einstellung mit Hilfe eines unter Ammoniak, Monoethanolamin, Diethanolamin, Triethanolamin, einem Alkali- oder Ammoniumkarbonat bzw. -hydrogenkarbonat oder einem Alkalihydroxid ausgewählten basischen Mittel eingestellt wurde, oder der pH-Wert mittels eines aus Salzsäure, Essigsäure, Milchsäure, Oxal- oder Borsäure ausgewählten Ansäuerungsmittels eingestellt wurde.

8. Zubereitung nach einem der Ansprüche 2 bis 7, dadurch **gekennzeichnet**, daß sie zusätzlich noch mindestens ein weiteres Reduktionsmittel enthält, das unter den folgenden Stoffen ausgewählt wurde: Thioglykolsäure, Glycerin- oder Glykolmonothioglykolat, Cysteamin und dessen acylierte C₁-C₄-Derivate, Cystein, N-Acetylcystein, N-Mercaptoalkylamiden von Zucker, β-Mercaptiopropionsäure und deren Derivate, Thiomilchsäure, Thioäpfelsäure, Pantethein, Thioglycerin, Alkali-oder Erdalkalisulfit oder-bisulfit, N-(Mercaptoalkyl)-ω-hydroxyalkylamide, N-Mono- oder N,N-4-Dialkylmercaptobutyramide, Aminomercaptoalkylamide, Derivate von sauren N-(Mercaptoalkyl)-succinamiden oder sauren Derivaten von N-(Mercaptoalkyl)-succinimiden.

9. Zubereitung nach einem der Ansprüche 2 bis 8, dadurch **gekennzeichnet**, daß sie zusätzlich noch mindestens ein kationisches Polymer, ein Erweichungsmittel, ein Proteinhydrolysat, ein Wachs, einen Opakmacher, ein Parfüm, einen Farbstoff, ein nichtionisches Tensid, einen Alkohol, ein Behandlungsmittel (Wirkstoff) oder auch noch ein Penetriermittel enthält.

10. Zubereitung nach einem der Ansprüche 2 bis 9, dadurch **gekennzeichnet**, daß sie zusätzlich noch mindestens ein Disulfid enthält, wobei die Zusammensetzung vom selbstneutralisierenden Typus ist.

11. Zubereitung nach Anspruch 10, dadurch **gekennzeichnet**, daß das Disulfid eine Dithioglykolsäure, ein Dithioglycerin, Cystamin, N,N'-Diacetylcystamin, Cystin, Pantethin oder ein N-(Mercaptoalkyl)-ω-hydroxyalkylamiddisulfid oder N-Mono- oder N,N-Dialkyl-4-mercaptobutyramid-disulfid, ein Aminomercaptoalkylamiddisulfid oder ein Disulfid von sauren N-(Mercaptoalkyl)-Succinamidderivaten oder von sauren N-(Mercaptoalkyl)-Succinimidderivaten ist.

12. Zubereitung nach Anspruch 11, dadurch **gekennzeichnet**, daß das Disulfit der folgenden allgemeinen Formel (III) entspricht: 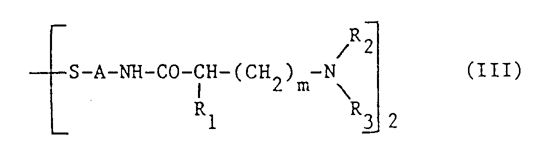 A, R₁, R₂ und R₃ sowie m die in Anspruch 1 im Hinblick auf die allgemeine Formel (I) angegebenen Bedeutungen aufweisen, jedoch mit Ausnahme einer Verbindung gemäß der Formel (III), worin
A = -(CH₂)₂-, m = 0
R₁ und R₂ = H und R₃ = -C₂H₅ bedeuten.

13. Zubereitung nach Anspruch 12, dadurch **gekennzeichnet**, daß das Disulfid gemäß der Formel (III) unter den folgenden Stoffen ausgewählt ist:
N,N'-(2,1-Dithiodiethandiyl)-bis-[2-dimethylaminoacetamid],
N,N'-(2,1-Dithiodiethandiyl)-bis-[2-dimethylaminopropionamid],
N,N'-(2,1-Dithiodiethandiyl)-bis-[3-dimethylaminopropionamid],
N,N'-(2,1-Dithiodiethandiyl)-bis-[4-dimethylaminobutyramid],
N,N'-(2,1-Dithiodiethandiyl)-bis-[2-methylaminoacetamid]
N,N'-[Dithiobis-(trimethylen)]-bis-[2-dimethylaminoacetamid]
N,N'-[Dithiobis-(pentamethylen)]-bis-[2-dimethylaminoacetamid]
N,N'-[Dithiobis-(2-ethoxyethyl)]-bis-[2-dimethylaminoacetamid]
N,N'-(2,1-Dithiodiethandiyl)-bis-[3-methylaminopropionamid],
N,N'-(2,1-Dithiodiethandiyl)-bis-[2-diethylaminoacetamid] sowie
N,N'-(2,1-Dithiodiethandiyl)-bis-[2-ethylaminoacetamid].

14. Zubereitung nach einem der Ansprüche 10 bis 13, dadurch **gekennzeichnet**, daß das Disulfid in einem molaren Verhältnis in bezug auf die Verbindung gemäß der Formel (I) von 0,5 bis 2,5, vorzugsweise von 1 bis 2, vorhanden ist.

15. Verfahren zur dauerhaften Verformung der Haare für eine Dauerwelle, das darin besteht, daß in einem ersten Arbeitsschritt zur Reduktion der Disulfidbindungen des Keratins eine reduzierende Zusammensetzung appliziert und im Anschluß daran in einem zweiten Schritt zur Wiederherstellung dieser Bindungen eine oxidierende Zusammensetzung appliziert wird, dadurch **gekennzeichnet**, daß der Reduktionsschritt mit Hilfe einer reduzierenden kosmetischen Zubereitung bewerkstelligt wird, wie sie nach einem der Ansprüche 2 bis 9 beansprucht ist.

16. Verfahren nach Anspruch 15, dadurch **gekennzeichnet**, daß man die reduzierende Zubereitung während einer Zeitdauer zwischen 5 und 60 Minuten einwirken läßt.
